(19) Europäisches Patentamt
European Patent Office
Office européen des brevets

(11) Publication number: 0 164 245 A2

# (12) EUROPEAN PATENT APPLICATION

(21) Application number: 85303750.5

(22) Date of filing: 29.05.85

(51) Int. Cl.⁴: C 12 N 15/00, C 12 N 1/20 // A01H1/00 ,(C12N1/20, C12R1:41)

(30) Priority: 08.06.84 US 618439

(43) Date of publication of application: 11.12.85 Bulletin 85/50

(84) Designated Contracting States: AT BE CH DE FR GB IT LI LU NL SE

(71) Applicant: Agrigenetics Research Associates Limited, 3375 Mitchell Lane, Boulder Colorado (US)

(72) Inventor: Hennecke, Hauke, Germaniastrasse 5, CH-8006 Zurich (CH)

(74) Representative: De Minvielle-Devaux, Ian Benedict Peter et al, CARPMAELS & RANSFORD 43, Bloomsbury Square, London WC1A 2RA (GB)

## (54) Nif promoter of rhizobium japonicum.

(57) There is disclosed a recombinant DNA plasmid comprising a vector and a promoter of a nitrogenase complex gene from a Rhizobium japonicum bacterium. The plasmid may additionally comprise a structural gene (which may be a gene from a Rhizobium species or a foreign gene) inserted in such orientation and location as to be expressible under control of the promoter.

Also disclosed are the nucleotide sequences of nifH and nifD promoters which may be used in the plasmid of the invention.

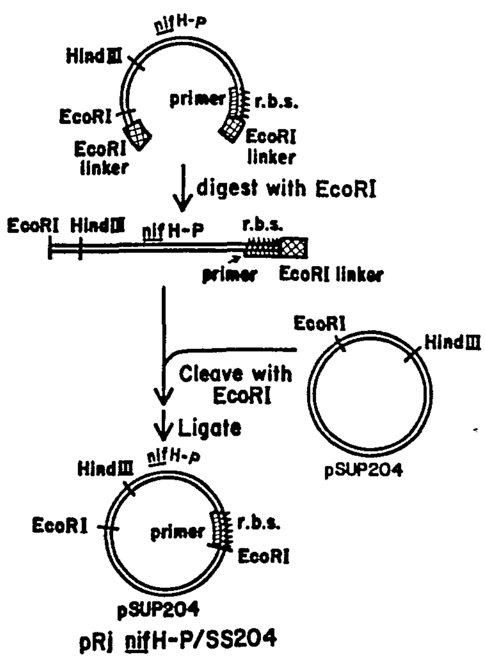


EP 0 164 245 A2

0164245



# NIF PROMOTER OF RHIZOBIUM JAPONICUM

## Field of the Invention

Biological nitrogen fixation in the root nodules of leguminous plants is a major component of world food production and therefore practical applications of this field are of major interest.

Prokaryotes can use a wide variety of nitrogen compounds as sole sources of cellular nitrogen. This variety includes ammonia, dinitrogen and nitrate among the inorganic compounds, and proline, arginine and glutamine among complex organic compounds. Each species can utilize a different array of nitrogen compounds. Glutamine, glutamate and aspartate are the key nitrogen compounds in intermediary metabolism. The latter two are the starting compounds of many pathways of amino acid biosynthesis and serve as amino group donors in many reactions. In all other cases the amino group is donated by glutamine. The major enzyme required for the assimilation of ammonia produced by $N_2$ fixation is glutamine synthetase, which catalyses the reaction:

Glutamate + $NH_3$ + ATP ----> glutamine + ADP + Pi.

At high $NH_4^+$ concentrations (> 1mM) glutamate dehydrogenase is also found. Utilization of the assimilated ammonia depends on the activity of glutamate synthase catalyzing:

Glutamine + 2-ketoglutarate + NADPH ----> 2 glutamate + $NADP^+$

Since ATP is hydrolysed, these reactions have a favorable equilibrium and allow the use of ammonia in the medium or ammonia derived enzymatically from other nitrogen sources (Meers, J., Tempest, D. and C. Brown (1970) J. Gen. Microbiol. 64:187-194). The formation of ammonia is thus a key step in the biological nitrogen cycle.

Biological nitrogen fixation can be achieved by a variety of microorganisms and occurs through the induction of an enzyme complex, nitrogenase, which converts atmospheric nitrogen to ammonia. This conversion

occurs in a group of physiologically diverse prokaryotes, including facultative anaerobes (e.g., Klebsiella pneumoniae and Rhodospirillum rubrum), obligate anaerobes (e.g., Clostridium pasteruianum), obligate aerobes (e.g., Azotobacter vinelandii) and some strains of blue-green algae (e.g., Anabaena cylindrica) (Sprent, J. I. (1979) The biology of nitrogen fixing organisms, London, McGraw-Hill, pp. 8-11). While this enzyme complex is common to all characterized nitrogen fixing organisms, the conditions under which it is expressed vary considerably between species (Burns, R. C., Hardy, R. W. F. (1975): Nitrogen fixation in bacteria and higher plants, Springer-Verlag, Berlin). The first stages of nitrogen fixation, conversion of nitrogen into ammonia, are achieved symbiotically in the root nodules of leguminous plants which contain the nitrogen-fixing bacteria of the genus Rhizobium. Some non-leguminous plants, e.g., alder, also have interactions with symbiotic bacteria which are nitrogen fixers. In addition, free-living bacteria, e.g., Klebsiella pneumoniae and the photosynthetic blue-green bacteria, also fix nitrogen. Biological nitrogen fixation in the root nodules of leguminous plants is a major component of world food production (Burris, R. H. (1980) In Free Living Systems and Chemical Models; Nitrogen fixation, Newton, W. E., Orne-Johnson, W. H., eds. Vol 1 Baltimore, University Park Press, pp. 7-16).

The symbiotic association between plants and bacteria of the genus Rhizobium is the result of a complex interaction between the bacterium and its host, requiring the expression of both bacterial and plant genes in a tightly coordinated manner (Vincent, J. M. (1980) In Symbiotic Associations and Cyanobacteria, Nitrogen Fixation Vol. 2 (W. E. Newton, W. H. Orne-Johnson, eds. Baltimore, University Park Press pp. 103-129; and Verma, D. P. S., Legocki, R. P. and S. Auger (1981) In Current Perspectives in Nitrogen Fixation (A. H. Gibson, W. E. Newton, eds.) Canberra: Australian Academy of Science, pp. 205-208). In free-living Rhizobia, nitrogenase synthesis is repressed and is only induced after the symbiotic relationship has been established. Furthermore, some Rhizobium species only interact with a narrow range of plant species, whereas other species interact with a wide range.

Bacteria bind to the emerging plant root hairs and invade the root tissue through the formation of an infection thread. The plant responds to this infection by the development of a highly differentiated root nodule. These nodules are the site of synthesis of the nitrogenase complex. Following

nitrogen fixation, the fixed nitrogen is exported into the plant tissue and assimilated by the plant derived enzymes (Scott, D. B., Farnden, K. J. F. and Robertson, J. G. (1976) Nature 263:703,705).

Most Rhizobium symbioses are confined to leguminous plants. Furthermore, Rhizobium strains which fix nitrogen in association with the agriculturally-important temperate legumes are usually restricted in their host range to a single legume genus. However, some strains of Rhizobium have been isolated which can fix nitrogen in a diverse group of legume species but can also form an effective symbiosis with non-legumes.

Despite the ability of certain plants to induce nitrogenase activity in a symbiotic relationship with some species of Rhizobium, the genetic analysis of biological nitrogen fixation has previously been confined to free living nitrogen fixing organisms, in particular Klebsiella pneumoniae. There are 17 linked nitrogen fixation (nif) genes arranged in at least 7 transcriptional units in the nif cluster of Klebsiella (Kennedy, C., Cannon, F., Cannon, M., Dixon, R., Hill, S., Jensen, J., Kumar, S., McLean, P., Merrick, M., Robson, R. and Postgate, J. (1981) In Current Perspectives in Nitrogen Fixation (A. H. Gibson, W. E. Newton, eds.) Canberra: Australian Academy of Science, pp. 146-156; and Reidel, G. E., Ausubel, F. M. and F. M. Cannon (1979) Proc. Nat. Acad. Sci. U.S.A. 76:2866-2870). Three of these genes, nifH, nifD and nifK encode the structural proteins of the nitrogenase enzyme complex (viz. the Fe-protein subunit (dinitrogenase reductase) and the α- and β-subunits of the Mo-Fe protein (dinitrogenase) respectively. Dinitrogenase is an $\alpha_2\beta_2$ tetramer in which the two non-identical α and β subunits have similar molecular weights of 55,000 to 60,000. Dinitrogenase reductase is a dimer of two identical subunits each having a molecular weight around 35,000. These genes are linked on the same operon in K. pneumoniae and are transcribed from a promoter adjacent to the nifH gene. A similar situation (nifHDK) was found in two fast-growing Rhizobia, R. meliloti (Ruvkun, G. B., et al. (1982) Cell 29:551-559) and R. leguminosarum (Schetgens, T. M. P. et al. (1984) Identification and analysis of the expression of Rhizobium leguminosarum PRE symbiotic genes, p. 699, In C. Veeger and W. E. Newton (eds.) Advances in nitrogen fixation research. Martinus Nijhoff/Dr. W. Junk Publishers, The Hague). In the slow-growing R. japonicum, it has been found that nifDK forms one operon and that nifH is located elsewhere on the genome (Fuhrmann, M. and H. Hennecke (1982)

Mol. Gen. Genet. 187:419-425). A similar observation was made with another member of the slow-growing rhizobia, Rhizobium sp. Parasponia: a nifH region was found not to be linked to nifD (Scott, K. F., et al. (1983) DNA 2:141-148). Yet a different arrangement was detected in the cyanobacterium Anabaena sp. 7120, in which nifHD is separated from nifK (Rice, D., et al. (1982) J. Biol. Chem. 257:13157-13163). The remainder of symbiotic genes contain information required for bacterial attachment, root hair curling, initiation and development of nodules and establishment of symbiotic relationships. In addition, regulatory sequences such as promoters, operators, attenuators, and ribosome binding sites are found adjacent to the coding regions. These regulatory sequences control the expression of the structural genes, i.e., the coding sequences downstream in the 3'-direction of the DNA reading strand.

The discovery and study of plasmids, restriction enzymes, ligases and other enzymes involved in DNA synthesis has led to the rapidly developing field of genetic engineering. Use of these techniques has made it possible to transfer DNA across species boundaries, either from eukaryotic to prokaryotic organisms or vice versa. Alternatively, it has been possible to synthesize nucleotide sequences and to incorporate these synthetic sequences into living organisms where they have been expressed. For example, expression in E. coli has been obtained with DNA sequences coding for mouse dihydrofolate reductase (Chang, A. C. Y., Nunberg, J. H., Kaufman, R. K., Ehrlich, H. A., Schimke, R. T. and Cohen, S. N. (1978) Nature 275:617-624) and for hepatitis B virus antigen (Burrell, C. J., Mackay, P., Greenaway, P. J., Hofschneider, P. H. and K. Murray (1979) Nature 279:43-47). Two mammal hormones have also been produced in bacteria by use of synthetic DNA (Itakura, K., Hirose, T., Crea, R., Riggs, A. D., Heynecker, H. L., Bolivar, F., and H. W. Boyer (1977) Science 198:1056; and Goeddel, D. B., Kleid, D. G., Bolivar, F., Heynecker, H. L., Yansura, D. G., Crea, R., Hirose, T., Kraszewski, A., Itakura, K. and A. D. Riggs (1979) Proc. Nat. Acad. Sci. U.S.A. 76:106).

The practical application of DNA recombination requires the success of a number of different features. First, it must be possible to recognize the DNA fragment coding for the compound of interest and it must be possible to isolate that DNA fragment. Second, it is necessary to understand the mechanisms which control the expression of the information on that DNA fragment and to be able to transfer that information to the control of

regulatory sequences which will maximize the productive capabilities of that information. This increased productive capacity could be by rearrangement of coding information and regulatory information within the same organism or between different organisms. The organisms involved may be prokaryotic or eukaryotic. Third, the conversion of coding information into useful products, such as storage proteins and hormones, must occur in an environment where they are not subsequently degraded.

## Background of the Invention

In bacteria of the genus Rhizobium, nitrogenase synthesis is normally repressed under free-living conditions and is induced only within a complex symbiosis formed mostly with leguminous plants. R. trifolii is an example of a fast-growing Rhizobium with a narrow host range and cannot normally be induced to fix nitrogen in culture. In contrast, a Parasponia Rhizobium species has been isolated and this species is a slow-growing organism with a very broad host range capable of an effective symbiotic relationship with a broad variety of tropical legumes as well as the non-legume Parasponia (Ulmaceae) (Trinick, M. J. (1980) J. Appl. Bacteriol. 49:39-53). Parasponia Rhizobium can be induced to fix nitrogen in culture although the level of this fixation is about 100-fold less than can be obtained from the free-living bacterium Klebsiella pneumoniae. Other slow-growing Rhizobia include the commercially significant R. japonicum, which nodulates soybeans.

The genetics of biological nitrogen fixation have been well characterized in the free-living organism Klebsiella pneumoniae. The structural genes for nitrogenase (nifH, nifD and nifK encoding the Fe-protein subunit and the α and β subunits of the Mo-Fe protein, respectively) have been mapped both genetically and physically (Kennedy, C. et al. (1981) In Current Perspectives in Nitrogen Fixation (eds. Gibson, A. H. and W. E. Newton) Australian Acad. Science, Canberra, pp. 146-156; and Reidel, G. E., Ausubel, F. M. and F. M. Cannon (1979) Proc. Nat. Acad. Sci. U.S.A. 76:2866-2870). Cloned DNA fragments carrying these sequences have been shown, by Southern blot analysis, to hybridize to homologous sequences in a wide range of nitrogen fixing organisms, including Rhizobium (Ruvkun, G. B. and F. M. Ausubel (1980) Proc. Nat. Acad. Sci. U.S.A. 77:191-195).

In spite of the ecological diversity of nitrogen fixing organisms, the physiological structure of the nitrogenase enzyme complex appears to be very conserved. In all cases where the enzyme complex has been purified, two proteins are present. The larger protein (dinitrogenase) contains molybdenum, iron and acid-labile sulfur, and carries the binding site for nitrogen and contains two subunit proteins α- and β-coded by the nifD and nifK genes respectively. The smaller protein (dinitrogenase reductase) contains iron and acid-labile sulfur, and is required for the reduction of the dinitrogenase and for the binding of MgATP used in this reduction. The dinitrogenase reductase is coded by the nifH gene. Chemical and spectral analyses of the purified protein components support a conservation of protein structure between organisms (Scott, K. F., Rolfe, B. G. and J. Shine (1981) J. Mol. Appl. Genet. 1:71-81). In some cases the structures are sufficiently similar to allow formation of active hybrid enzymes between purified components, e.g., Azotobacter vinelandii and Klebsiella pneumoniae (Eady, R. R. and B. E. Smith (1979) In: A treatise on dinitrogen fixation I, II, eds. Hardy, R. W., Bottomley, F. and R. C. Burns, New York, Wiley Press pp. 399-490). Not surprisingly, therefore, the region of the nif operon coding for dinitrogenase reductase and dinitrogenase α-subunit (nifH and nifD) shows homology at the nucleic acid sequence level with the corresponding sequences in at least 19 other bacterial strains (Ruvkun, G. B. and F. M. Ausubel (1980) Proc. Nat. Acad. Sci. U.S.A. 77:191-195). Although this conservation of structure is generally true, significant differences between nitrogenases from different organisms also exist as can be shown by variable stability following purification and by the fact that active hybrid complexes do not form in all cases (Eady, R. R. and B. E. Smith (1979) supra).

A DNA fragment carrying the Klebsiella pneumoniae nifK, nifD and nifH genes has been isolated from the $nif^-$ strain UNF841(Tn5::nifK) (Cannon, F.C. et al. (1979) Mol. Gen. Genet. 174:59-66) and cloned into the Escherichia coli plasmid pBR325. The nucleotide sequences of the nifH gene and of 622 nucleotides of the nifD gene were determined (Sundaresan, V. and F. M. Ausubel (1981) J. Biol. Chem. 256:2808-2812; Scott, K. F., Rolfe, B. G. and J. Shine (1981) supra). In addition, the DNA sequence of the nifH gene from Anabaena 7120 has been determined (Mevarech, M., Rice, D. and R. Haselkorn (1980) Proc. Nat. Acad. Sci. U.S.A. 77:6476-6480). A comparison of the two sequences demonstrates two interesting features: (1) There is very little homology

between the two sequences at the nucleotide sequence level although a few stretches (up to 25bp) are conserved, accounting for the observed interspecies homology of the nif genes (Ruvkun, G. B. and F. M. Ausubel (1980) supra); (2) In general, the promoter regions show very little sequence homology with the exception of a short region likely to be involved in common functions, e.g., RNA polymerase recognition.

In contrast, a comparison of the amino acid sequences of the dinitrogenase reductase and of the first 207 amino acids of the α-subunit of dinitrogenase of the two species and of another species show a much greater conservatism. The three species used in this comparison are Klebsiella pneumoniae (Kp); Anabaena 7120 (Ab); and Clostridium pasteurianum (Cp) (Tanaka, M., Haniu, M., Yasunobu, T. and L. Mortenson (1977) J. Biol. Chem. 252:7093-7100). The Kp and Cp proteins share 67% amino acid sequence homology, Kp and Ab proteins share 71% homology, and the Cp and Ab proteins share 63%. This amino acid sequence homology is not spread evenly throughout the protein. Some regions are virtually identical--90% to 95% homology), while other regions are only weakly conserved (30-35% homology). The structural conservation appears to be centered around the five cysteine residues common to all three Fe proteins. These cysteine residues are believed to be ligands to the active center.

Comparison of the N-terminal amino acid sequence of the α-subunit of dinitrogenase from Cp and Kp shows very little sequence homology in this region. This is in contrast to the very high conservation of amino acid sequence seen in the amino terminal region of the Fe protein. What little homology exists between Cp and Kp α-subunits is confined to regions around cysteine residues, as in the Fe proteins. These homologous regions are thought to be involved in the catalytic functions of the nitrogenase enzyme complex. Therefore, this structural conservatism is thought not to be the result of recent evolution and dispersal of the nif genes (Postgate, J. R. (1974) Sym. Soc. Gen. Microbiol. 24:263-292) but, rather, is postulated to be related to a conservation of function.

Summary of the Invention

A recombinant plasmid is disclosed, wherein there is a wide host range vector containing an inserted fragment of DNA including a promoter of a nitrogenase complex gene of Rhizobium japonicum and a foreign structural gene under the control of the regulatory region. Since the regulatory region and the foreign genes are carried on plasmids which can be lost from transformed Rhizobium japonicum, a method for transferring these genes from a vector to the bacterial chromosome is also described. Novel Rhizobium japonicum strains are thereby generated, having a chromosomally integrated composite gene including a nitrogenase gene regulatory region and a foreign gene.

Detailed Description of the Invention

Rhizobium japonicum, a member of the slow-growing group of rhizobia or "bradyrhizobia" (Jordan, D. C. (1982) Int. J. Syst. Bacteriol. 32:136-139) is an important soybean symbiont and is therefore most important in the agricultural industry. The structural organization of the nif genes differs from "fast-growing" Rhizobia in that the nifH and nifDK genes which code for the polypeptides of the nitrogenase complex, have been shown to be unlinked (Kaluza, K. et al. (1983) J. Bacteriol. 155:915-918). The fix complex is here defined to include all genes involved in the fixation of nitrogen in a Rhizobium japonicum/host plant symbiosis. The interesting question was raised as to whether or not the expression of these genes was coordinately regulated and whether there were homologies in the DNA control regions. The three nitrogenase structural genes have previously been cloned (Hennecke, H. (1981) Nature (London) 291:354-355). The polypeptides of dinitrogenase were expressed in E. coli (Fuhrmann, M. and H. Hennecke (1982) Mol. Gen. Genet. 187:419-425); the nifD gene product (α-subunit) was shown to possess a molecular weight of 58,000, whereas the nifK gene product (β-subunit) has a molecular weight of 55,000. The nifD and nifK genes are organized on a transcriptional unit, and are transcribed in the direction nifDK. Interestingly, no other nif genes were found in the immediate vicinity on either side of nifDK. The sources of DNA were the R. japonicum nifDK-containing recombinant plasmid pRJ676 and subclones thereof (Hennecke, H. (1981) supra) and the nifH containing recombinant plasmid pRJ7000 and subclones thereof. Polypeptides encoded by these various R. japonicum

recombinant plasmids were analysed using the polypeptide products synthesized in mini cells of E. coli. DNA sequencing analysis was done using both the chemical method (Maxam, A. M. and W. Gilbert (1980) Methods Enzymol. 65:449-561) and the chain termination method (Sanger, F. et al. (1977) Proc. Nat. Acad. Sci. U.S.A. 74:5463-5467). All restriction endonuclease sites shown in the figures were confirmed by overlapped sequencing. Both DNA strands of the genes and flanking sequences were sequenced. Transcriptional start points and the extent of the mRNA transcripts were determined by nuclease S1 mapping (Berk, A. J. and P. A. Sharp (1977) Cell 12:721-732).

Seven base pairs (bp) upstream from the ATG translation start site of the R. japonicum nifD gene (Fig. 1) there is a 5'-AGGA-3' sequence marking a perfect ribosome binding site (Shine, J. and L. Dalgarno (1974) Proc. Nat. Acad. Sci. U.S.A. 71:1342-1346; Stormo, G. D., et al. (1982) Nucleic Acids Res. 10:2871-2996). A similar sequence 5'-TGGA-3 (Fig. 2) occurs eight bp upstream from the ATG translation start site of the nifH operon. In the case of the nifD gene, including and following the ATG codon, there is an open reading frame of 1545 bases which can be translated into 515 amino acids. This open reading frame is terminated by three consecutive stop codons (TGA, TAG, TGA) and immediately adjacent to the 3'-side of the last stop codon is another ribosome binding site (5-AGGA-3') with a spacing of eight bp in front of the ATG start site of the nifK gene (Fig. 1). Only the first 132 bases of the nifK gene have been sequenced but these can be translated into 44 amino acids. Twenty three of these amino acids are directly homologous to corresponding amino acid positions in the amino terminal portion of the Anabaena nifK gene product (Mazur, B. J. and C. F. Chui (1982) Proc. Nat. Acad. Sci. U.S.A. 79:6782-6786).

The utilization of the triplet codons of the R. japonicum nifD gene (Fig. 3) show that two codons are not used. Most of the other codons are used in an asymmetric or strongly asymmetric manner (e.g., those for Phe, Asn, Gly and Cys). The codon usage for the sequenced 622 bp stretch of Klebsiella pneumoniae nifD DNA (Scott, K. F. et al. (1981) J. Mol. Appl. Genet. 1:71-81) is substantially different. In the case of R. japonicum, when more than two triplets are available for a given amino acid, there is a preference of using those with a G and/or C in the "wobble" position. This could reflect the G + C content of slow-growing Rhizobium species.

Previous results had suggested that there was no further nif gene on the 5'-flanking side of nifD as part of a common operon (Kaluza, K. et al. (1983) J. Bacteriol. 155:915-918). The 5'-nifD flanking sequence was therefore examined for the presence of transcription initiation sites. RNA both from free-living, $N_2$-fixing R. japonicum cells as well as from soybean root nodule bacteroids was hybridized to a $^{32}P$-labelled HpaII fragment (see Examples) and the hybrid was digested with nuclease S1 (Berk, A. J. and P. A. Sharp (1977) Cell 12:721-732). The start point of transcription was determined by electrophoresis of the S1-protected DNA adjacent to a "Maxam-Gilbert" sequencing ladder of the same HpaII fragment: it was found to be 46 nucleotides (± 1 nucleotide) (Brosius, J. et al. (1982) J. Biol. Chem. 257:9205-9210) upstream from the ATG codon of nifD (marked by an arrow in Fig. 4).

The area in front of the transcriptional start does not contain any sequences which are reminiscent of typical E. coli RNA polymerase binding sites (Rosenberg, M. and D. Court (1979) Ann. Rev. Genet. 13:319-353). However, the nifDK promoter (a promoter is here defined as the nucleotide sequence upstream from the transcriptional start site containing all the regulatory regions required for transcription) has a sequence 5'-CTGG-8bp-TTGCA starting 26 nucleotides before the transcription start site. (A translational start site is here defined as the ATG-codon translated into a methionine residue at the amino terminus of an open-reading frame and a transcription start site is the first deoxynucleotide to be transcribed into a mRNA sequence.) This promoter sequence unexpectedly showed similarities to a promoter sequence of Klebsiella pneumoniae nif genes (Beynon, J. et al. (1983) Cell 34:665-671). (Since K. pneumoniae is a species which does not participate in the bacterial-plant symbiotic relationships for nitrogen reduction, the factors which regulate the activities of the nif genes are likely to be quite different. Beynon et al. proposed that the TTGCA sequence was equivalent to a "Pribnow box" of genes regulated by nitrogen control (Drummond, M. et al. (1983) Nature (London) 301:302-307; Beynon, J. et al. (1983) Cell 34:665-671; Ow, D. W., et al. (1983) Proc. Nat. Acad. Sci. U.S.A. 80:2524-2528) and the CTGG sequence may be the recognition target for nif-activating proteins (Beynon, J. et al. (1983) supra). However, the operation of the present invention is not dependent upon the correctness of these proposals.

The complete nucleotide sequence of the R. japonicum nifH gene is presented (Fig. 2) together with the predicted amino acid sequence of the nifH gene product (the dinitrogenase reductase). From the presumptive start codon (ATG) to the stop codon (TAA) there is an open reading frame of 882 nucleotides corresponding to 294 amino acids. Eight nucleotides upstream from the start codon there is a ribosome binding site, 5'-TGGA-3'.

The transcriptional start point of the R. japonicum nifH gene was determined as described (supra). RNA both from free-living, $N_2$-fixing R. japonicum cells and from soybean root nodule bacteroids was hybridized to a $^{32}P$-labelled HindIII/HinfI fragment which extends from position -201 of the 5'-nifH flanking region to nucleotide +41 of the coding region (see Fig. 2). The hybrid was digested with nuclease S1, and the protected DNA was electrophoresed adjacent to a Maxam-Gilbert "sequencing ladder" of the same HindIII/HinfI fragment. The transcription start point was thus determined to be the G located 153 nucleotides upstream from the beginning of the nifH coding region (marked by an arrow in Fig. 2). The transcription start point is immediately preceded by the promoter region (for definition, see above) containing the sequence 5'-TTGG-8bp-TTGCT-3'. (Note: the corresponding sequence of the nifDK promoter given above is 5'-CTGG-8bp-TTGCA-3'.) Furthermore, the region from positions -185 to -160 (Fig. 2) contains 19 nucleotides found at identical positions in the promoter region of the R. japonicum nifDK operon (see Fig. 4 for comparison of R. japonicum nifDK and nifH).

The region flanking the 3'-end of the R. japonicum nifH coding region was also examined for termination signals. A very obvious inverted repeat structure was found 13 nucleotides downstream from the last nifH codon (Fig. 2). Messenger RNA transcribed from this region could potentially form a characteristic stem and loop structure:

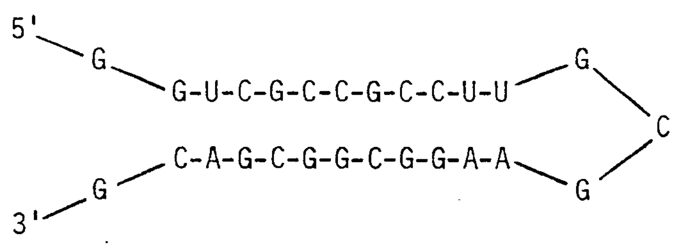

The stem is formed by 11 consecutive base pairs of which eight are G-C pairs. This structure is quite stable with a free energy of $\Delta G°$ (25°C) = -26.2 kcal estimated according to Tinoco *et al*. (Tinoco, J., Jr. *et al*. Nature New Biology 246:40-41). On the 3'-flanking side the inverted repeat structure is followed by a C-rich region.

The postulated G/C-rich terminator region is of the "*E. coli*" type, except that the stem and loop structure is followed by a C-rich region rather than a T-rich region (Rosenberg, M. and D. Court (1979) Ann. Rev. Genet. 13:319-353). The postulated ribosome binding site is also similar to those of *E. coli* genes (Shine, J. and L. Dalgarno (1974) Proc. Nat. Acad. Sci. U.S.A. 71:1342-1346) which may explain why it has been possible to translate *nif*H fusion mRNA into (a) *nif*H-specific polypeptide(s) in *E. coli* minicells (see Example 8 and Fig. 5). These translation experiments have revealed a protein doublet with molecular weights of 33,000 and 32,000 encoded by the same DNA region. At present it is unknown whether this reflects protein processing or the presence of two overlapping open reading frames. Indeed, 12bp upstream from the *nif*H coding region, and in frame with it, there is a second possible ATG initiator codon, but this second codon is not preceded by a characteristic Shine-Dalgarno sequence (Fig. 2). It is interesting to note that a similar doublet of the *K. pneumoniae* dinitrogenase reductase was found *in vivo* (Roberts, G. P. *et al*. (1978) J. Bacteriol. 136:267-279), and that the expression of the *R. meliloti nif*H gene has also led to the synthesis of two proteins with similar molecular weights (Weber, G. and A. Puhler (1982) Plant. Molec. Biol. 1:305-320).

The *nif*H promoter sequence of *R. japonicum* differs from the *nif*DK promoter region in only a few base pairs. Both promoter sequences contain a characteristic region between nucleotide 9 and 25 upstream from the transcription start site

$$5'\text{-}\binom{C}{T}\text{TGGCA}\binom{T}{C}\text{G}\binom{C}{G}\text{C}\binom{G}{T}\text{GTGC}\binom{A}{T}\text{-}3'$$

with some similar features of the *K. pneumoniae nif* promoters (Beynon, J. *et al*. (1983) Cell 34:665-671). However, with respect to the *nif*H and *nif*DK promoters of *R. japonicum* there are nine additional identical nucleotides (5'-GTGC-5bp-AGACC-3') immediately preceding (i.e., upstream) these sequences. These sequences (i.e., 5-GTGC-5bp-AGACC-3') could be involved in the correct

functioning of the promoters. As some of these elements are likely to be involved in nif gene regulation, it is surprising to observe what may be remnants of nif gene control circuits in the non-symbiotic K. pneumoniae which appear to operate in the agriculturally important symbiotic R. japonicum. When DNA sequences are compared, then, in addition to the similarities with the promoter elements of Klebsiella pneumoniae (Sundaresan, V. et al. (1983) Nature (London) 301:728-732; Beynon, J. et al. (1983) Cell 34:665-671; Ow, D. W. et al. (1983) Proc. Nat. Acad. Sci. U.S.A. 80:2524-2528), the nifH and nifDK promoters of Rhizobium japonicum have some homology with the nifH promoter from Rhizobium sp. Parasponia (Scott, K. F. et al. (1983) DNA 2:141-148) and with the nifH promoter (P1) from Rhizobium meliloti (Sundaresan, V. et al. (1983) Nature (London) 301:728-732; Better, M. et al. (1983) Cell 35:479-485). However, it should be noted that the regions of non-homology are likely to be most significant because the species which can be infected by Rhizobium japonicum are quite different from those species which can be infected by either Rhizobium sp. Parasponia or Rhizobium meliloti. Thus the latter two species cannot be utilized in any gene expression under nif gene promoter control when it is desirable to use soybeans as the host species in a bacterium-plant symbiotic relationship.

Since the promoter regions of the nifH operon and of the nifDK operon have been isolated, characterized and cloned, it is possible to delete the nifH and nifDK nitrogenase genes, i.e., the DNA sequences normally transcribed into mRNA or RNA, and replace them with (a) structural gene(s) isolated from an extraneous source organism ("foreign gene" herein). The foreign gene(s) thus placed under the control of the nifH and nifDK promoters can then be inserted into a plasmid vector followed by conjugation into Rhizobium japonicum. A vector is defined here as a plasmid with a single replication origin which carries and replicates one or more fragments of foreign DNA. The foreign gene is then expressed in this novel R. japonicum under conditions where the nif gene promoters are activated. Alternatively, the novel composite gene which includes the foreign structural gene and the nif gene promoter, can be integrated with the chromosome of a host R. japonicum in order to maximize the stability of the trait conferred by the composite gene.

The novel plasmids disclosed herein are useful for amplifying the quantities of composite genes, for transferring such genes to selected R.

japonicum host strains, for generating new R. japonicum host strains and as intermediates for the construction of plasmids having one or more of the foregoing uses. The R. japonicum strains of the present invention are useful for expressing the composite gene, under certain conditions, to provide a useful product, to confer an advantageous property to a plant or to improve the rate, quality or efficiency of the foreign gene product. In particular, the properties of the novel strains are manifested within root nodules formed by novel R. japonicum strains of the invention in symbiotic combination with a host plant. Depending upon the foreign gene chosen for expression in the nodule, the nodule then serves as a production source for a protein coded by the foreign gene. Examples of proteins which can be expressed in root nodules include the insect-toxic protein of Bacillus thuringiensis (Wong, H. C. et al. (1983) J. Biol. Chem. 258:1960 ff.), the hydrogenase found in some but not all Rhizobium strains (Cantrell, M. et al. (1983) Proc. Nat. Acad. Sci. U.S.A. 80:181 ff.), metallothionein (Karin, M. and R. I. Richards (1982) Nucleic Acids Res. 10:3165 ff.) and prolactin (Cooke, N. et al. (1981) J. Biol. Chem. 256:4007-4016). The foregoing list is not intended as limiting, but merely as an exemplary of the broad range of possibilities for synthesis of proteins in root and stem nodules of plants. In general, the invention makes it possible to produce any protein that may be of use, either as a product extracted from the nodule, as an excretion product of the nodule, conferring an advantage for the host plant, or as a functional protein within the nodule itself, improving the effectiveness of the symbiotic interaction. In addition to the proteins disclosed herein, others will be apparent to those of ordinary skill in the art, taking advantage of the known or subsequently discovered properties of root nodules and of specific proteins. A major advantage conferred by gene expression under control of a nif regulatory region in root nodules is derived from the inventor's recognition that such expression is regulated in a similar manner as the expression of the nif genes themselves. The foreign gene is only minimally expressed, if at all, in the free living bacteria but is maximally expressed within the root nodule. Furthermore, because of the specific nature of the host-bacterium symbiosis, gene expression occurs only in the selected plant species recognized by the modified bacterial strain. These properties consequently insure, first, that the foreign gene expression provides maximum local effect of the expression product, and second, that environmental side effects are limited since gene expression can be confined to the nodular tissue of the selected soybean plant strain or variety.

Expression of Foreign Genes Downstream from the nifH and nifDK Promoters of Rhizobium japonicum

A principle feature of the present invention is the construction of a plasmid having an inserted foreign gene under control of a nifH or nifDK promoter. The structural gene must be inserted in the correct position and orientation with respect to the promoter in order to obtain expression of the structural gene controlled by the promoter. Position has two aspects. The first relates to which side of the promoter the structural gene is inserted. It is known that the majority of promoters control initiation of transcription and translation in one direction only along the DNA. The region of DNA lying under promoter control is said to lie "downstream" or alternatively on the 3'-side of the promoter. Therefore, to be controlled by the promoter, the correct position of the foreign gene insertion must be "downstream" from the promoter. The second aspect of position refers to the distance, in base pairs, between functional elements of the promoter, for example, the transcription initiation site and the translational start site of the foreign gene. Substantial variation appears to exist between promoters with respect to this distance. Therefore the structural requirements in this regard are best described in functional terms. Optimum spacing can be achieved by experiments varying the length of this distance. As a first approximation, reasonable operability can be obtained when the distance between the promoter and the inserted foreign gene is similar to the distance between the promoter and the gene it normally controls. Orientation refers to the directionality of the structural gene. By convention, that portion of a structural gene which ultimately codes for the amino terminus of a protein is termed the 5' end of the structural gene, while that end which codes for amino acids near the carboxyl end of a protein is termed the 3' end of the structural gene. Correct orientation of a structural gene is with the 5' end thereof proximal to the promoter. An additional requirement in the case of constructions leading to fusion protein expression is that the insertion of the structural gene into an existing nitrogenase complex structural gene sequence must be such that the coding sequences of the two genes are in the same reading frame phase, a structural requirement which is well understood in the art.

In order to express foreign genes in the 3'-side of the nitrogenase complex regulatory sequences, it is first advantageous to construct a doublestranded DNA sequence corresponding to the nifH or nifD regulatory sequences. To achieve this, synthetic DNA primer complementary to the ribosome binding site of the m-RNA and extending a few nucleotides to the side thereof is first constructed. Then the cloned nifH or nifD fragment is excised from the vector, purified and the excised nifH or nifD fragments are ligated into appropriate single stranded DNA phage (e.g., fd) vectors. The resultant recombinant DNA plasmids are then transformed into E. coli strains, and single colonies are propagated. Those colonies which extrude single stranded templates corresponding to the m-RNA strand are isolated. The synthetic DNA is used as a primer on these single stranded templates to generate double stranded DNA by primer extension with DNA polymerase I (Klenow fragment). This double stranded DNA will extend from the ribosome binding site to an indeterminate point within the single stranded DNA vector. Any single stranded regions are removed by S1 nuclease treatment. Alternatively, a double stranded vector, e.g., pBR322, may be denatured and replicated using the same synthetic DNA primer. If a double stranded vector is used, then suitable precautions well known to those skilled in the art should be used to avoid the presence of background unlabelled fragments, e.g., it is possible to demonstrate the presence of contaminating fragments by use of restriction maps.

Then synthetic EcoRI linkers are ligated to the DNA fragments followed by digestion with EcoRI and that restriction endonuclease (termed endonuclease A for generality) which recognizes the restriction site at the 5' end of the nifH or nifD. The resultant DNA fragments are then cloned into an EcoRI-endonuclease A cleaved plasmid, transformed into a suitable E. coli host and amplified. The choice of plasmid is based on principles of operating convenience and location of the appropriate restriction sites, as will be understood by those of ordinary skill in the art.

Following amplification, isolation and repurification, this same plasmid is then cleaved with endonuclease A and treated with S1 nuclease or BAL-31 for a short time to produce blunt ended fragments. The plasmid is now cleaved with EcoRI and the fragment is cloned into the wide host range plasmid pRK290 to produce a pRK290-nif regulatory fragment construct. Alternatively, another

wide host range plasmid, pSUP204, can be used to construct the recombinant *nif* regulatory plasmid.

Alternatively, the DNA fragments provided with *EcoRI*-endonuclease A-specific ends are initially cloned into a mobilizable broad host range vector capable of replication in either *E. coli* or most other gram-negative bacteria, such as pSUP104 or pSUP204 (R. Simon *et al*., 'Vector plasmids for *in vivo* and *in vitro* manipulations of gram-negative bacteria' in Molecular Genetics of the Bacteria-Plant Interaction (A. Puhler, Ed.), Springer-Verlag, Heidelberg (1983)).

In order to clone and express foreign genes, appropriate DNA fragments carrying these foreign genes are isolated and synthetic *EcoRI* linkers are ligated to the fragments (*EcoRI*-foreign gene-*EcoRI*). These *EcoRI*-foreign gene-*EcoRI* DNA fragments are then ligated into EcoRI-cleaved vector DNA, for example, pSUP104 or pSUP204, resulting in a *nif*-regulated expression plasmid, pSS104 or pSS204, respectively, and transformed into *Escherichia coli*. After selection and amplification, the *nif*-regulated expression plasmid is then transferred with the aid of helper plasmids to the appropriate *Rhizobium* or *Agrobacterium* strain by mating.

The exconjugant *Rhizobium* strains are then used to infect soybean plants or other appropriate legumes which are subsequently assayed for the production of foreign mRNA and/or protein.

## Introduction of DNA Sequences Into the Chromosome/Genome of Gram-Negative Organisms Other Than E. coli

Plasmids are lost rather easily from bacterial strains, thus leading to the loss of expression of those genes carried on the plasmids. One method of stabilizing the expression of certain genes carried on plasmids, or, for that matter, any foreign DNA segment, would be the introduction of such genes or foreign DNA segments, hereinafter termed "introduced DNA", into the chromosome of the host bacteria. Such a system employs a "suicide vector" and, preferably, a transposon.

Suicide vectors are plasmid molecules which replicate stably in one bacterial host (in this case, *Escherichia coli*) but fail to replicate in a different bacterial species (e.g., *Rhizobium trifolii*).

Transposons are genetic elements which are able to move (translocate) from one location to another in DNA. The translocation process is mediated by gene products encoded on the transposon and is dependent upon the integrity of repeated sequences (directly or indirectly repeated) located at each end of the transposon. Transposons generally carry a gene (or genes) encoding resistance to one (or more) antibiotics. The transposon and the suicide vector are linearized and religated into a single recombinant DNA molecule.

The general method of transferring introduced DNA segments to the chromosome of a gram-negative bacterial strain other than E. coli is outlined here. The DNA fragments to be introduced can be generated in a number of ways: (a) by restriction with site-specific restriction endonucleases; (b) by partial or complete digestion with restriction endonucleases which generate DNA fragments having blunt ends; (c) by digestion of DNA with the enzyme DNAase I in the presence of $Mn^{2+}$ ions thus generating random fragments which are generally blunt-ended; or (d) by shearing the DNA into large fragments.

In the preferred method, the suicide vector carrying a transposon with an antibiotic resistance gene is linearized and the appropriate fragment of introduced DNA is ligated into a "co-integrated recombinant molecule". The fragment of DNA is inserted into a restriction endonuclease site within the transposon in such a manner that the insertion does not disrupt normal transposition nor expression of the drug resistance marker. This ligated DNA is then transformed into an E. coli strain in which it can be amplified and mobilized for transfer into other gram-negative bacteria.

The cloned, introduced DNA fragment from this E. coli strain can then be moved into the chromosome of any gram-negative bacterium, e.g., Rhizobium japonicum. This is most conveniently achieved by the process of bacterial conjugation. The E. coli strain carrying the suicide vector which contains an antibiotic resistance gene, is mixed with cells of the antibiotic sensitive gram-negative strain on the surface of a nutrient agar plate. The plate is incubated for a period (4-16 hours) at the optimum temperature of the gram-negative strain and, during this time, cells of each bacterial species come into physical contact (conjugation) and the suicide vector is transferred from the donor E. coli to the recipient gram-negative strain. The cell mixture is washed off the plate and spread on an agar plate which is selective for the antibiotic resistance. It is preferred to include selection means that select

against growth of the E. coli parent strain once the conjugation and transfer is completed.

Since the suicide vector containing the introduced fragment of DNA cannot be amplified autonomously in the recipient gram-negative strain, a transfer of genetic material to the bacterial chromosome can occur in one of three ways: (a) If a fragment of the recipient gram-negative bacterial chromosome (BC) has been previously inserted into the suicide vector (SV) thus creating a region of homology between the suicide vector and the recipient gram-negative bacterial chromosome, then a single reciprocal recombination will result in the incorporation or cointegration of the entire recombinant molecule into the chromosome of the recipient gram-negative bacterial chromosome (Fig. 6). The boxed area indicates the position of insertion of the introduced gene fragment and $D^R$ indicates the position of the antibiotic resistance gene. (b) If a fragment of the recipient gram-negative bacterial chromosome has been previously inserted into the suicide vector thus creating a region of homology between the suicide vector and the recipient gram-negative bacterial chromosome and then an introduced DNA fragment and a drug resistance gene are inserted into this region of homology, a double reciprocal recombination event will incorporate only the introduced DNA fragment and the drug resistance gene into the chromosome of the recipient gram-negative bacterial strain (Fig. 7). Such recombination is site-specific, the chromosomal location being determined by the fragment of chromosomal DNA carried on the suicide vector. The components of the figures are labelled as shown (Fig. 7). (c) In the preferred method, the transposon containing an introduced DNA fragment and an antibiotic resistance gene may be transposed into the bacterial chromosome of the recipient gram-negative bacterial strain (Fig. 8). The components of the figure are labelled as shown (Fig. 8). In addition, Tn refers to a transposon used to transpose the inserted DNA into the bacterial chromosome. Selection for the antibiotic resistance ensures maintenance of the inserted DNA.

For the sake of convenience and clarity, definitions used are gathered together and presented below:

(a) Nitrogenase complex: all genes involved in the fixation of nitrogen in a Rhizobium japonicum host plant symbiosis.

(b) Promoter: the nucleotide sequence upstream from the transcriptional start site containing all the regulatory regions required for transcription.

(c) Translational start site: the ATG codon translated into a methionine residue at the amino terminus of an open reading frame.

(d) Transcription start site: the first deoxynucleotide to be transcribed into an RNA sequence of an mRNA sequence.

(e) Foreign gene(s): (a) structural gene(s) isolated from an extraneous source organism.

(f) Vector: a plasmid with a single replication origin which carries and replicates one or more fragments of foreign DNA.

(g) Foreign DNA: a fragment of DNA isolated from an extraneous source organism.

(h) Stringent conditions: incubation at 50°C for 3 hours.

Example 1: Source of DNA and DNA sequencing

For nifDK, the source of DNA was the R. japonicum nifDK-containing recombinant plasmid pRJ676, and subclones thereof (Hennecke, H. (1981) Nature (London) 291:354-355; Fuhrmann, M. and H. Hennecke (1982) Mol. Gen. Genet. 187:419-425). For nifH, a previous Southern blot hybridization experiment with total DNA of Rhizobium japonicum had revealed that a PstI fragment of about 12-14 kilobase pairs (kb) specifically hybridizes to radioactively labelled nifH DNA from Klebsiella pneumoniae and Rhizobium meliloti. In order to clone this fragment, size-fractionated R. japonicum DNA (PstI fragments between 10 and 16 kb) was ligated with PstI-digested DNA of the cloning vector pHE3, and the recombinant DNA was transformed into E. coli RR28 (Hennecke, H. et al. (1982) Gene 19:231-234). As this cloning system enables the direct selection of recombinant plasmids, 730 transformants were picked and screened for the presence of the R. japonicum nifH gene by interspecies colony hybridization under stringent conditions. Stringent conditions are defined here as incubation at 50°C for 3 hours. One colony (pRJ7000) contained the desired recombinant plasmid with a 12.1kb insert that hybridized to both the ($^{32}$P)-labelled nifH-containing R. meliloti HindIII fragment of pRmR2 (Ruvkun, G. B. and F. M. Ausubel (1980) Proc. Nat. Acad. Sci. U.S.A. 77:191-195), and to the ($^{32}$P)-labelled nifH containing K. pneumoniae EcoRI/KpnI fragment of pSA31 (Scott, K. F., et al. (1981) J. Mol. Appl. Genet. 1:71-81) (see also Fig. 5 for a restriction enzyme cleavage map of the 12.1 kb R. japonicum fragment of pRJ700). The region of nifH homology is indicated by a dotted line.

Nucleotide sequence data were obtained using both the chemical method as well as the chain termination method. Chemical sequencing was done by the methods of Maxam and Gilbert (Maxam, A. and W. Gilbert (1980) Methods Enzymol. 65:499-561) except that the A + G modification procedure was done as described by Gray et al. (Gray, C. P. et al. (1978) Proc. Nat. Acad. Sci. U.S.A. 75:50-53). For the chain termination method (Sanger, F. et al. (1977) Proc. Nat. Acad. Sci. U.S.A. 74:5463-5467), DNA fragments were subcloned into the bacteriophages M13mp7, -mp8, -mp9, -mp10 and -mp11 (Messing, J. (1983) Methods Enzymol. 101:20-78). After transformation of E. coli JM103, recombinant phages were screened using the dot-blot hybridization technique. Single stranded DNA was isolated as described (Messing, J. (1983) Methods Enzymol.

101:20-78) and annealed to primers purchased from Bethesda Research Laboratories (26 bases) or New England Biolabs (15 bases). All restriction sites were confirmed by overlapped sequencing, and the complete nifD (Figs. 1 and 9) and nifH regions were sequenced on both strands of DNA. The nifK region was partially sequenced (Figs. 1 and 9). A unique and, as yet, unexplained feature is the fact that DNA fragments to the 5' side of the nifD coding region are not cloneable in M13 vectors, and had to be sequenced by the chemical method. Because of this feature, it was not possible to use phage M13 to construct a double stranded DNA sequence corresponding to the nifH or nifD regulatory sequences. However, other single stranded phage vectors (e.g., fd) could be used for these constructs. A computer program was used for analysis and storage of the sequence data (Larson, R. and J. Messing (1982) Nucleic Acids Res. 10:39-49).

Example 2: Determination of promoter and terminator regions by S1 nuclease mapping

The transcriptional start points of the *R. japonicum* nifH and nifD genes were determined by nuclease S1 mapping (Berk, A. J. and P. A. Sharp (1977) Cell 12:721-732) with modifications (Weaver, R. F. and C. Weissmann (1979) Nucleic Acids Res. 7:1175-1192). In the case of nifD the source of RNA was free-living, nitrogen fixing cultures of *R. japonicum* strain 110spc4 (Regensburger, B. and H. Hennecke (1983) Arch. Microbiol. 135:103-109), or 110spc4 bacteroids from soybean (Glycine max L. Merr. var. Clark L-1) nodules, isolated as described (Sundaresan, V. et al. (1983) Nature (London) 301:728-732). RNA was extracted as described previously (Kaluza, K. and H. Hennecke (1981) Arch. Microbiol. 130:38-43). RNA from free-living, aerobic *R. japonicum* cultures served as a negative control. For nifD, the 5'-$^{32}P$-labelled DNA fragment was used as a 145 bp HpaII fragment extending from nucleotide positions -63 to +82 (Fig. 1). For nifH, a 5'-$^{32}P$-labelled HindIII/HinfI fragment extending from position -201 to +41 of the coding region was used (Fig. 2). After strand separation and hybridization to mRNA (45°C for 12 hours) unhybridized nucleic acids were digested with nuclease S1 (Boehringer Mannheim), and the protected DNA was electrophoresed adjacent to a Maxam-Gilbert "sequencing ladder" of the same HpaII fragment (nifD) or HindIII/HinfI fragment (nifH).

Example 3: Expression of foreign genes under the control of a nifH promoter DNA region. Method I.

Construct a synthetic DNA primer which is complementary to the ribosome binding site of the Rhizobium japonicum nifH gene (5'-GCTGCTCTCCATCAACCG-3'). A DNA fragment which spans the region from an EcoRI restriction site upstream (i.e., 5'- to the ATG translation initiation codon) to a XhoII site downstream (i.e., 3'- to the ATG translation initiation codon) is then subcloned into a single stranded DNA phage, transformed into E. coli JM103 (Fig. 10) and propagated therein. The cloned fragment is amplified and single stranded templates (ca. 1μg) are recovered from the supernatant following centrifugation of the bacterial host. A 10-fold excess of the synthetic DNA primer in the presence of the four deoxynucleotide triphosphates (one of which is radioactive) and DNA polymerase I (Klenow fragment) is now used as a primer on this nifH template to generate double stranded DNA (dsDNA) (Fig. 10). The mixture is incubated for 15-45 minutes at 25° to 37°C during which time the complementary strand is substantially extended. The remaining single stranded DNA is then removed by digestion with S1 nuclease (Fig. 10). EcoRI linkers (GGAATTCC) are then ligated to the double stranded DNA fragments followed by digestion with EcoRI (Fig. 11). The fragments are separated by agarose gel electrophoresis and the fragment containing the promoter sequence is eluted and cloned into the wide host range plasmid pSUP204 (Fig. 11), which has previously been restricted by the restriction enzyme EcoRI. The resulting recombinant plasmid is termed pRj-nifH-P/SS204. Following transformation and amplification in a suitable E. coli host strain, e.g., 17-1 which is restriction negative, i.e., $r^-$, partial cleavage with EcoRI allows the addition of any foreign structural gene or foreign DNA fragment into the linearized plasmid downstream from the nifH promoter fragment. For example, the human prolactin gene can be inserted (Cooke, N. et al. (1981) J. Biol. Chem. 256:4007-4016) or the human metallothionein gene can be inserted (Karin, M. and R. I. Richards (1982) Nucleic Acids Res. 10:3165-3173 and see Example 5) resulting in a "composite" recombinant. A composite recombinant is herein defined as a recombinant DNA plasmid containing a vector, a promoter sequence and any foreign DNA whose expression is under the control of said promoter sequence.

Example 4: Expression of foreign genes under the control of a nifD promoter DNA region. Method I.

Construct a synthetic DNA primer which is complementary to the ribosome binding site of the Rhizobium japonicum nifD gene (5'-CTGGTGTCCTTTAAAAG-3'). The nifD specific BglII/ClaI fragment which spans the region -375 to +732 and includes the nifD promoter (nifD-P) is then subcloned into a single stranded DNA phage, e.g., fd, transformed into E. coli JM103 (Fig. 12) and incubated in 0.1M salt at 30°C. Alternatively the nifD specific BglII/ClaI fragment can be cloned into pBR322. Following amplification in a suitable host, the recombinant DNA is purified and a radioactive DNA primer with the same sequence (see above) used to prime the double stranded DNA synthesis and to recognize the appropriate fragment following electrophoresis. Methods well known in the art (e.g., restriction maps) are used to check the purity of the isolated, radioactive fragment. The cloned fragment is amplified and single stranded templates corresponding to the mRNA strand of R. japonicum nifD are packaged and extruded into the media. The single stranded templates (ca. 1μg) are recovered from the supernatant following centrifugation of the bacterial host. A 10-fold excess of the synthetic DNA primer in the presence of the four deoxynucleotide triphosphates (one of which is radioactive) and DNA polymerase I (Klenow fragment) is now used as a primer on this nifD template to generate dsDNA (Fig. 12). The mixture is incubated for 15-45 minutes at 25° to 37°C during which time the complementary strand is substantially extended. The remaining single stranded DNA is then removed by digestion with S1 nuclease (Fig. 12). EcoRI linkers (GGAATTCC) are then ligated to the double stranded DNA fragments followed by digestion with EcoRI and XhoII (Fig. 13). The fragments are separated by agarose gel electrophoresis and the 1.1 kilobase pair fragment is eluted and cloned into the wide host range plasmid pSUP204 (Fig. 13), which has previously been restricted by the restriction enzyme BamHI, conversion of the restriction site to a blunt end, and finally digestion with EcoRI. The resulting recombinant plasmid is termed pRjnifD-P/SS204. Following transformation and amplification in a suitable E. coli host strain, e.g., 17-1, cleavage with EcoRI allows the addition of any foreign structural gene or foreign DNA into the linearized plasmid. For example, the human prolactin gene can be inserted (Cooke, N. et al. (1981) J.

Biol. Chem. 256:4007-4016) or The human metallothionein gene can be inserted (Karin, M. and R. I. Richards (1982) Nucleic Acids Res. 10:3165-3173 and see Example 5) resulting in a co-integrated recombinant.

Example 5: Insertion of the human metallothionein gene into the recombinant plasmids pRjnifH-P/SS204 or pRjnifD-P/SS204

The procedure followed in this example is the same as that followed in Examples 3 and 4 up to the point where EcoRI linkers are ligated to the dsDNA fragments followed by digestion with EcoRI for pRjnifH-P/SS204 (Fig. 11). Only the experimental procedure for nifH-P is diagrammed since the procedures are very similar. For nifD-P, XhoII can be read in place of HindIII (compare Figs. 11 and 13). The resultant DNA fragment (approximately 551 base pairs for nifH-P) is then cloned into EcoRI (nifH-P) cleaved pBR322 (Fig. 14). Following transformation and amplification in a suitable *E. coli* host strain, the recombinant plasmids are partially cleaved with EcoRI (nifH-P) (Fig. 14) and treated with S1 nuclease for a short time to remove the overhangs. The recombinants are then cleaved with EcoRI (Fig. 14) and the double stranded nif regulatory fragments are cloned into EcoRI cleaved pRK290 DNA (see Fig. 15 for details). The resulting recombinants are thus pRK290-nif-promoter fragment constructs (pRK290-nifH-P or pRK290-nifD-P). pRK290 is a wide host range plasmid.

The next step is to isolate DNA fragments carrying the foreign genes of interest and to ligate synthetic EcoRI linkers to these fragments. These modified fragments are then ligated into EcoRI cleaved vector DNA (i.e., the pRK290-nif promoter fragment constructs) giving a "composite" recombinant (pRK290-nif promoter fragment-foreign gene) (Fig. 15) and transformed into an *E. coli* host strain, e.g., 17-1 or RR1. The composite recombinant is then transferred to *Rhizobium japonicum* by bacterial conjugation using a helper plasmid whenever necessary. The *R. japonicum* bacteria carrying the composite recombinant are then used to infect plants and later assayed for the production of foreign mRNA and/or protein by standard methods known in the art.

Example 6: Insertion of the bacterial toxin gene from Bacillus thuringiensis into the recombinant plasmid pRjnifH-P/SS204

Recombinant plasmids containing inserts of the gene encoding the toxic crystal protein of B. thuringiensis are obtained using the techniques described (Wong, H. C., Schnepf, H. E. and H. R. Whiteley (1983) J. Biol. Chem. 258:1960-1967). The recombinant plasmid pES1 (ATCC Number 31995) consisting of the plasmid vector pBR322 and DNA homologous to the 30, 32 and 37 megadalton plasmids, as well as DNA homologous to linearized forms of the very large plasmids of B. thuringiensis is partially cleaved with EcoRI to give linear molecules. These partial cleavage products are further restricted by the enzyme AvaI. The digestion conditions are as recommended by the manufacturer. A probe for the toxic crystal protein gene is isolated and radioactively labelled as previously described (Wong, H. C. et al. (1983) supra). The restriction fragments are separated by agarose gel electrophoresis and the labelled probe is found to hybridize to one fragment of approximately 15 kilobases (kb). This fragment includes the EcoRI fragments D and F (Wong, H. C. et al. (1983) supra). The 15 kb fragment is then cloned into M13mp8 or M13 mp9 according to standard procedure (Messing, J. and J. Vieira (1982) Gene 19:269-276) and transformed into E. coli JM103. The single stranded DNA from the extruded phage particles is purified and replicated in vitro by use of a synthetic primer (5'-TGTTATCCA-TGGGTTACCTCC-3') (The general method of site specific mutagenesis is described in Zoller, M .J. and M. Smith (1982) Nucleic Acids Research 10:6487-6500). The resulting double stranded recombinant plasmid is then transformed back into E. coli JM103 and amplified. The amplified double stranded plasmid DNA is purified from the E. coli JM103 cells and cleaved with the restriction endonucleases NcoI and AvaI. NcoI cleaves at the site of the synthetic primer (which is the initiation site of the toxic crystal protein gene) and AvaI cleaves at a site which is downstream from the 3'-end of the toxic crystal protein gene. The overhangs are then filled in to blunt ends (Maniatis, T., Jeffrey, A. and D. G. Kleid (1975) Proc. Nat. Acad. Sci. U.S.A. 72:1184-1188).

Finally the pRj-nifH-P/SS204 recombinant plasmid which is derived from pSUP204 (Fig. 11) is cleaved with EcoRI and the overhangs filled in to blunt ends. HindIII linkers are then added to both the B. thuringiensis toxic crystal protein gene fragment and to the pRjnifH-P/SS204 recombinant.

Following the HindIII digestion of both components, the toxic crystal protein gene and the pRjnifH-P/SS204 recombinant plasmid are ligated together to give a pRjnifH-P/SS204 - B. thuringiensis toxic crystal protein gene composite. The mixture is transformed into a suitable E. coli host, e.g. K802, SM10 or RR1. Plasmids are isolated from individual colonies and the orientation determined by restriction mapping. A colony containing a plasmid with the correct orientation is the conjugated to Rhizobium japonicum and the plasmid is transferred as already described (Examples 3 and 4). The production of mRNA and/or the toxic crystal protein is monitored as already described (Wong, et al., supra).

Example 7: Introduction of DNA sequences into the genome of gram-negative organisms other than E. coli

This example is based on the following general principles. Two basic components are required. These are: (1) a suicide vector, and (2) a transposon.

Suicide vectors are plasmid molecules which replicate stably in one bacterial host (in this case, Escherichia coli) but fail to replicate in a different bacterial species (e.g., Rhizobium japonicum).

Transposons are genetic elements which are able to move (translocate) from one location to another in DNA. The translocation process is mediated by gene products encoded on the transposon and is dependent upon the integrity of repeated sequences (directly or indirectly repeated) located at each end of the transposon. Transposons generally carry a gene (or genes) encoding resistance to one (or more) antibiotics.

In the protocol to be outlined below, use is made of the transposon designated Tn5 and the suicide vector pSUP1011 (Simon, R., Priefer, U. and A. Puhler (1981) Proc. of Bielefeld Symposium, Springer-Verlag, West Germany) (see Fig. 16).

Transposon Tn5 is a DNA element of 5.7 kilobases (kb) in length, consisting of 1.5kb inverted repeat sequences flanking a 2.7 kb central region. Encoded within one of the inverted repeats are the functions required for transposition. The central region of the transposon carries a gene conferring resistance to the antibiotic kanamycin ($Km^r$). In the middle of the

central region is a DNA sequence which is recognized by the restriction endonuclease BamHI. In the suicide vector pSUP1011, the only site recognized and cut by BamHI is that located within the Tn5 element. Experiments (Simon, R., Priefer, U. and A. Puhler (1983) Proc. of Bielefeld Symposium, Springer-Verlag, West Germany) have shown that insertion of DNA fragments into the BamHI site of Tn5 does not disrupt normal transposition nor expression of the kanamycin-resistance gene of the resultant "hybrid" transposon.

The DNA fragment to be introduced can be generated in a number of ways:

1) Complete or partial restriction with BamHI, Sau3A, MboI, etc. which generate fragments having the same, complementary, single-stranded ends.

2) Partial or complete digestion with restriction endonucleases which generate DNA fragments having blunt ends.

3) Digestion of DNA with the enzyme DNAaseI in the presence of $Mn^{++}$ ions which generates random fragments which (generally) are blunt ended.

The suicide vector (pSUP1011) DNA is treated as follows depending on the type of fragment to be cloned (above):

1) Complete restriction with endonuclease BamHI and treatment with the enzyme alkaline phosphatase.

2) Complete restriction with BamHI followed by either:

a) treatment with S1 nuclease to remove the single-stranded ends, or

b) "filling in" of the single-stranded ends by the enzyme reverse transcriptase in the presence of nucleotide triphosphates.

Each of the above treatments is followed by treatment with alkaline phosphatase.

Cloning: Vector and fragment DNA, prepared as above, are mixed and treated with the enzyme T4 DNA ligase. The ligated DNA is then transformed

(introduced) into Escherichia coli strain SM10. (This strand is capable of mobilizing ($Mob^+$) pSUP1011 derivatives (recombinant plasmids) into other gram-negative bacteria.) (Simon, R., Priefer, U. and A. Puhler (1983) Proc. of Bielefeld Symposium, Springer-Verlag, West Germany). The resultant transformants are screened by the Grunstein and Hogness colony hybridization procedure (Grunstein, M. and D. S. Hogness (1975) Proc. Nat. Acad. Sci. U.S.A. 72:3961) to detect those containing the desired cloned DNA fragment.

Introduction of the cloned DNA fragment into the genome of any gram-negative bacterium (e.g., Rhizobium japonicum) is achieved via a process called bacterial conjugation. The E. coli SM10 derivative, carrying the desired pSUP1011 recombinant, is mixed with cells of (kanamycin-sensitive) R. japonicum on the surface of a nutrient agar plate. The plate is incubated for a period (4-16 hours) at 29-30°C (optimum temperature for R. japonicum) and during this time cells of each type come into physical contact (conjugation) and the pSUP1011 derivative is transferred from E. coli to R. japonicum. The cell mixture is washed off the plate and spread on an agar plate which is selective for kanamycin-resistant R. japonicum. The resultant colonies will be derivatives of R. japonicum in which the cloned DNA fragment, within Tn5, will be inserted at some point in the genome. Selection for kanamycin resistance ensures maintenance of the inserted DNA.

At this stage it is unknown whether the DNA fragment, within Tn5, has been transferred to the chromosome of R. japonicum or to one of its several plasmids. This uncertainty can be resolved by visualization of the plasmids and the bacterial chromosome by ethidium bromide staining after horizontal agarose gel electrophoresis (Djordjevic, M. A., Zurkowski, W. and B. G. Rolfe (1982) J. Bacteriol. 151:560-568).

Example 8: Expression of the nitrogenase nifH gene in E. coli minicells

The polypeptides encoded by the R. japonicum nifH region were analysed in minicells of E. coli. For this purpose a nifH-containing EcoRI fragment was cloned into the chloramphenicol resistance gene of pACUC184 in both possible orientations to give pRJ7003 and pRJ7004 (Fig. 5). Expression was thus mediated by the strong promoter of the gene for chloramphenicol acetyltransferase. pRJ7003 coded for the synthesis of two proteins with molecular weights of 33,000 and 32,000. In the opposite orientation (pRJ7004)

a 20,000 molecular weight protein was made indicating the presence of a long, open reading frame in that direction. Tn5 insertion derivatives of pRJ7003 were constructed and they were tested to see which of the mutations exerted a polar effect on the expression of the encoded proteins. The 33/32 K doublet was synthesized by the mutant plasmid pRJ7003::TN5-31, but not by pRJ7003::Tn5-22 or pRJ7003::Tn5-45 (Fig. 5) indicating that the 33/32 K proteins are encoded by the DNA region between the Tn5 insertions numbers 45 and 31, whereby the polar effects of insertions numbers 45 and 22 define the direction of transcription (Fig. 5).

CLAIMS

1. A recombinant DNA plasmid comprising

(a) a vector, and

(b) a promoter of a nitrogenase complex gene from a *Rhizobium japonicum* bacterium.

2. A recombinant DNA plasmid as recited in claim 1 wherein said vector is pRK290 or pSUP204.

3. A recombinant DNA plasmid as recited in Claim 1/ or Claim 2 wherein said promoter is a nifH promoter.

4. A recombinant DNA plasmid as recited in Claim 3 wherein said nifH promoter comprises the nucleotide sequence 5'-T-T-T-G-G-C-T-G-T-T-G-G-C-G-T-T-C-A-T-G-T-T-T-G-C-G-A-T-T-G-T-T-T-G-T-T-C-G-T-T-G-T-C-T-G-A-C-A-G-C-C-G-G-G-C-A-G-A-T-C-T-T-G-T-C-A-G-A-T-C-C-A-A-A-A-C-A-G-C-C-T-A-C-G-A-T-C-G-C-G-C-G-C-C-G-G-C-T-G-G-T-T-G-C-T-T-T-T-G-G-A-A-A-C-G-T-A-A-T-C-A-G-A-A-G-C-T-T-A-A-G-G-T-G-C-C-G-G-G-T-T-A-G-A-C-C-T-T-G-G-C-A-C-G-G-C-T-G-T-T-G-C-T-G-A-T-A-A-G-C-G-G-C-A-G-C-A-A-C-A-C-T-G-A-G-T-G-A-G-G-G-C-T-G-A-G-T-G-C-A-C-G-C-C-G-A-C-G-T-G-T-A-A-G-G-C-G-A-G-C-G-A-T-G-C-G-C-T-C-C-T-T-C-C-C-T-T-G-A-A-C-C-C-G-T-G-T-G-C-C-C-C-C-G-T-T-T-C-T-G-C-G-A-G-G-G-A-A-G-C-A-A-A-G-C-T-C-G-C-A-A-A-A-G-A-A-G-C-G-C-G-C-A-A-C-G-T-T-G-G-C-A-A-A-T-C-G-G-T-T-G-A-T-G-G-A-G-A-G-C-A-G-C-3', or a functionally equivalent sequence hybridizable thereto under stringent conditions.

5. A recombinant DNA plasmid as recited in Claim 1/ or Claim 2 wherein said promoter is a nifD promoter.

6. A recombinant DNA plasmid as recited in Claim 5 wherein said nifD promoter comprises the nucleotide sequence 5'-G-A-T-C-T-A-G-C-G-C-G-C-G-A-G-A-T-C-G-C-A-G-T-A-C-A-T-T-A-C-A-T-C-G-C-A-G-G-T-T-G-A-G-C-A-C-C-T-G-T-A-G-G-T-T-T-C-A-A-T-A-A-G-T-A-T-G-A-A-G-G-A-T-T-G-G-A-A-G-C-C-A-G-A-C-T-T-G-C-G-G-C-C-T-G-C-T-T-C-C-G-C-C-T-T-A-C-A-C-C-G-C-A-G-A-G-C-G-T-T-G-C-T-G-T-C-G-C-G-C-A-T-A-A-A-C-T-C-A-A-A-A-A-C-T-C-C-G-C-C-G-T-G-A-A-T-T-T-C-A-C-G-C-G-C-G-G-C-C-G-C-C-T-T-G-T-T-C-C-A-T-T-T-C-G-A-C-A-C-C-G-C-C-A-A-A-C-C-A-A-G-A-G-T-C-C-C-T-C-G-C-A-A-A-G-G-C-G-A-G-T-G-T-C-G-G-A-T-T-C-G-C-A-A-C-A-A-C-A-G-C-C-C-G-T-C-A-C-C-G-T-A-C-A-A-G-T-C-G-C-G-C-T-A-A-G-A-A-A-C-T-G-T-T-G-T-T-G-T-T-C-T-A-G-T-T-T-T-A-G-T-G-C-T-C-A-T-G-A-G-A-C-C-C-T-G-G-C-A-T-G-C-C-G-G-T-T-G-C-A-A-A-G-T-C-T-T-G-G-A-T-C-A-A-G-A-A-G-C-C-G-C-C-C-T-C-C-C-A-A-G-A-G-C-T-A-A-C-C-T-T-T-T-A-A-A-G-G-A-C-A-C-C-A-G-3' or a functionally equivalent sequence hybridizable thereto under stringent conditions.

7. A recombinant DNA plasmid as recited in any preceding Claim comprising additionally a structural gene inserted in such orientation and location as to be expressible under control of said promoter.

8. A recombinant DNA plasmid as recited in Claim 7 wherein said structural gene is obtained from a Rhizobium species.

9. A recombinant DNA plasmid as recited in Claim 7 wherein said structural gene is obtained from R. trifolii or R. meliloti.

10. A recombinant DNA plasmid as recited in Claim 7 wherein said structural gene is obtained from Parasponia Rhizobium.

11. A recombinant DNA plasmid as recited in Claim 7 wherein said structural gene is a foreign gene.

12. A recombinant DNA plasmid as recited in Claim 7 wherein said structural gene is a human prolactin gene or a human metallothionein gene.

13. A recombinant DNA plasmid as recited in Claim 7 wherein said structural gene is a toxin gene.

14. A recombinant DNA plasmid as recited in Claim 13 wherein said toxin gene is a bacterial toxin gene of Bacillus thuringiensis.

15. A bacterial strain containing and replicating therein a recombinant DNA plasmid according to any of claims 7 to 14.

16. A method for expressing a structural gene under control of a promoter c a nitrogenase complex gene from a Rhizobium japonicum bacterium comprising the steps

(a) isolating said promoter of a nitrogenase complex gene,

(b) cloning said promoter of a nitrogenase complex gene into a wide host range plasmid producing a recombinant DNA plasmid,

(c) isolating a DNA fragment carrying foreign structural genes and inserting said DNA fragment into said recombinant DNA plasmid at a position on the 3'-side of said promoter of a nitrogenase complex reading strand giving a co-integrated recombinant plasmid, wherein said DNA fragment is oriented with respect to said promoter of said nitrogenase complex gene as to be expressible under control thereof,

(d) transforming said co-integrated recombinant plasmid into a Rhizobium japonicum strain capable of a symbiotic relationship with plant cells, and

(e) infecting a soybean plant with said Rhizobium japonicum strain wherein expression of mRNA or protein coded by said foreign structural gene occurs.

17. A method as recited in Claim 16, wherein the product of step (c) is a recombinant DNA plasmid as recited in any of claims 7 to 14.

18. A method for incorporating a DNA sequence into the chromosome of a Rhizobium japonicum other than Escherichia coli comprising the steps

(a) isolating a DNA fragment comprising the sequence to be incorporated,

(b) inserting said DNA fragment into a transposon carried on a suicide vector, said transposon having a selectable resistance gene contained within said transposon, to give a recombinant DNA plasmid,

(c) transforming said recombinant DNA plasmid into a first strain of bacteria capable of mobilizing said suicide vector into said Rhizobium japonicum,

(d) transferring said recombinant DNA plasmid from said first strain of bacteria to said chromosome of said Rhizobium japonicum by recombination following bacterial conjugation,

(e) growing said Rhizobium japonicum under conditions requiring the presence of said selectable resistance gene, and

(f) selecting colonies of said Rhizobium japonicum obtained from step (e) wherein said DNA fragment comprising the sequence to be incorporated is recombined with said chromosome of said Rhizobium japonicum.

19. A method as recited in Claim 18 wherein said DNA fragment is derived from a genome of a species of the genus *Agrobacterium*.

20. A method as recited in Claim 18 wherein said DNA fragment is derived from a genome of a species of the genus *Rhizobium*.

21. A method as recited in Claim 20 wherein said species of the genus *Rhizobium* is *Rhizobium trifolii*.

22. A method as recited in Claim 20 wherein said species of the genus *Rhizobium* is *Parasponia Rhizobium*.

23. A method as recited in any of Claims 18 to 22 wherein said suicide vector is pSUP1011.

24. A method as recited in any of Claims 18 to 23 wherein said transposon with said selectable resistance gene is transposon Tn5 with a kanamycin resistance gene.

25 A method as recited in any of Claims 18 to 24 wherein said strain of bacteria capable of mobilizing said suicide vector is *Escherichia coli* SM10.

# FIG. 1

GATCTAGCGCGCGAGATCGCAGTACATTACATCGCAGGTTGAGCACCTGTAGGTTTCAATAAGTATGAAGGATTGGAAG
-350 -300

CCAGACTTGCGGCCTGCTTCCGCCTTACACCGCAGAGCGTTGCTGTCGCGCATAAACTCAAAAACTCCGCCGTGAATTT
-250

CACGCGCGGCCGCCTTGTTCCATTTCGACACCGCCAAACCAAGAGTCCCTCGCAAAGGCGAGTGTCGGATTCGCAACAA
-200 -150

CAGCCCGTCACCGTACAAGTCGCGCTAAGAAACTGTTGTTGTTCTAGTTTTAGTGCTCATGAGACCCTGGCATGCCGGT
-100

Met Ser Leu Ala Thr
TGCAAAGTCTTGGATCAAGAAGCCGCCCTCCCAACAGCTAACCTTTTAAAGGACACCAG ATG AGT CTC GCC ACG
-50 1

Thr Asn Ser Val Ala Glu Ile Arg Ala Arg Asn Lys Glu leu Ile Glu Glu Val Leu Lys
ACC AAC AGC GTC GCA GAA ATC ACG GCT CGC AAC AAA GAG CTG ATC GAG CAG GTG CTG AAG
50

Val Tyr Pro Glu Lys Thr Ala Lys Arg Arg Ala Lys His Heu Asn Val His Gln Ala Gly
GTC TAT CCG GAG AAA ACC GCG AAA AGG CGT GCC AAG CAC CTC AAC GTG CAC CAA GCA GGT
100

Lys Ser Asp Cys Gly Val Lys Ser Asn Ile Lys Ser Ile Pro Gly Val Met Thr Ile Arg
AAG TCG GAC TGC GGG GTG AAG TCC AAC ATC AAA TCC ATA CCC GGC GTG ATG ACG ATA AGA
150

Gly Cys Ala Tyr Ala Gly Ser Lys Gly Val Val Trp Gly Pro Ile Lys Asp Met Val His
GGG TGC GCC TAT GCA GGG TCG AAG GGG GTG GTC TGG GGA CCA ATC AAG GAC ATG GTT CAT
200 250

Ile Ser His Gly Pro Val Gly Cys Gly Gln Tyr Ser Trp Gly Ser Arg Arg Asn Tyr Tyr
ATT AGC CAT GGC CCG GTT GGC TGC GGC CAA TAT TCA TGG GGC TCG CGG CGC AAC TAT TAC
300

Val Gly Thr Thr Gly Ile Asp Ser Phe Val Thr Leu Gln Phe Thr Ser Asp Phe Gln Glu
GTT GGC ACC ACG GGC ATC GAT AGC TTC GTG ACT CTG CAG TTC ACC TCG GAC TTC CAG GAA
350

Lys Asp Ile Val Phe Gly Gly Asp Lys Lys Leu Asp Lys Ile Leu Asp Glu Ile Gln Glu
AAG GAT ATC GTA TTT GGC GGC CAC AAG AAA CTG GAC AAA ATC CTT GAT GAA ATC CAA GAG
400

Leu Phe Pro Leu Asn Asn Gly Ile Thr Ile Gln Ser Glu Cys Pro Val Gly Leu Ile Gly
CTG TTT CCA CTC AAC AAC GGC ATT ACG ATA CAA TCA GAG TGC CCG GTA GGC TTG ATC GGT
450

# FIG. 1-2

Asp Asp Ile Glu Ala Val Ser Arg Ala Lys Ser Lys Glu Tyr Gly Gly Lys Thr Ile Val
GAC GAT ATC GAG GCG GTG TCA AGG GCG AAA TCC AAA GAA TAT GGA GGC AAG ACC ATC GTG
500 550

Pro Val Arg Cys Glu Gly Phe Arg Gly Val Ser Gln Ser Leu Gly His His Ile Ala Asn
CCG GTC CGT TGT CAG GGC TTT CGG GGT GTG TCG CAG TCA CTA GGC CAT CAC ATT GCA AAC
600

Asp Ala Val Arg Asp Trp Ile Phe Gly His Ile Glu Ala Glu Gly Lys Pro Lys Phe Glu
GAT GCG GTA CGC GAT TGG ATT TTC CGG CAT ATC GAG GCC GAG CCC AAA CCA AAG TTC GAG
650

Pro Thr Pro Tyr Asp Val Ala Ile Ile Gly Asp Tyr Asn Ile Gly Gly Asp Ala Trp Ser
CCG ACA CCA TAC GAT GTT GCG ATC ATC GGA GAC TAC AAT ATC GGC GGC GAT GGT TGG TCA
700

Ser Arg Ile Leu Leu Glu Glu met Gly Leu Arg Val Ile Ala Gln Trp Ser Gly Asp Gly
TCG CGA ATT CTG CTT GAA GAG ATG GGA CTA CGG GTA ATC GCG CAG TGG TCC GGC GAC GGT
750

Ser Leu Ala Glu Leu Glu Ala Thr Pro Lys Ala Lys Leu Asn Ile Leu His Cys Tyr Arg
TCA CTG GCC GAG CTC GAA GCA ACG CCG AAG GCA AAG CTC AAG ATT CTG CAT TGC TAC CGT
800 850

Ser Met Asn Tyr Ile Ser Arg His Met Glu Glu Lys Phe Gly Ile Pro Trp Cys Glu Tyr
TCC ATG AAC TAT ATC TCA CGC CAC ATG GAA GAG AAG TTC GGC ATC CCT TGG TGC GAG TAC
900

Asn Phe Phe Gly Pro Ser Lys Ile Ala Asp Ser Leu Arg Arg Ile Ala Gly Tyr Phe Asp
AAC TTC TTC GGA CCT TCA AAG ATC GCG GAC TCA CTG CGC AGG ATT GCG GGT TAT TTT GAC
950

Asp Lys Ile Lys Glu Gly Ala Glu Arg Val Ile Glu Lys Tyr Gln Pro Leu Val Asp Ala
GAC AAG ATC AAG GAA GGC GCC GAG CGA GTG ATC GAG AAG TAT CAG CCG CTG GTG CAC GCC
1000

# FIG. 1-3

```
Val Ile Ala Lys Tyr Arg Pro Arg Leu Glu Gly Lys Thr Val Met Leu Tyr Val Gly Gly
GTG ATT GCA AAA TAT CGC CCG CGC CTC GAG GGC AAG ACG GTG ATG CTG TAC GTC GGC GGC
                     1050

Leu Arg Pro Arg His Val Ile Gly Ala Tyr Glu Asp Leu Gly Met Asp Val Ile Gly Thr
CTT CGT CCG CGT CAT GTG ATT GGC GCG TAC GAG GAC CTC GGG ATG GAC GTC ATT GGC ACT
    1100                                                                1150

Gly Tyr Glu Phe Gly His Asn Asp Asp Tyr Gln Arg Thr Ala Gln His Tyr Val Lys Asp
GGC TAC GAG TTC GGT CAC AAC GAC GAC TAT CAG CGC ACA GCT CAG CAC TAC GTG AAG GAC
                                                            1200

Ser Thr Leu Ile Tyr Asp Asp Val Asn Gly Tyr Glu Phe Glu Arg Phe Val Glu Arg Leu
AGC ACC CTC ATC TAT GAT GAC GTC AAT GGC TAT GAG TTC CAG CGC TTC GTC GAA AGA CTC
                                                1250

Gln Pro Asp Leu Val Gly Ser Gly Ile Lys Glu Lys Tyr Val Phe Gln Lys Met Ser Val
CAG CCT GAT CTT GTC GGC TCA GGC ATC AAG GAA AAG TAC GTT TTC CAA AAG ATG AGT GTG
                                1300

Pro Phe Arg Gln Met His Ser Trp Asp Tyr Ser Gly Pro Tyr His Gly Tyr Asp Gly Phe
CCG TTC CGG CAG ATG CAT TCG TGG GAC TAT TCG GGT CCA TAT CAC GGT TAT GAC GGC TTT
                     1350

Ala Ile Phe Ala Arg Asp Met Asp met Ala Val Asn Ser Pro Ile Trp Lys Arg Thr Lys
GCG ATC TTC GCG CGC GAC ATG GAC ATG GCC GTC AAC TCG CCA ATT TGG AAA AGA ACG AAA
    1400                                                                1450

Ala Pro Trp Lys Asp Ala Glu Arg Gln Asp Ser Arg Leu Gln Asn Asn Ala Thr Arg Leu
GCT CCC TGG AAG GAC GCC GAG CGC CAA GAC TCC AGG CTG CAG AAT AAC GCA ACT CGT CTC
                                                            1500

Ala Leu Arg Glu Ser Pro Gly Ile Pro Ile                         Met Pro Gln Ser
GCT CTC CGG GAA AGC CCG GGG ATT CCA ATC TGA TAG TGAAGGATTTCACCG ATG CCG CAG AGT
                                                1550

Ala Glu His Val Leu Asp His Val Glu Leu Phe Arg Gly Pro Glu Tyr Gln Gln Met Leu
GCC GAA CAC GTG CTC GAT CAT GTC GAA CTG TTC CGC GGT CCA GAA TAC CAG CAA ATG CTG
                            1600

Ala Lys Lys Lys Ile Phe Glu Asn Pro Arg Asp Pro Ala Glu Val Glu Arg Ile Lys Glu
GCG AAG AAG AAG ATA TTC GAG AAT CCG CGC GAT CCT GCC GAG GTC GAA CGT ATC AAG GAA
                 1650
```

# FIG. 2-1

```
(5')...TTTGGCTGTTGGCGTTCATGTTTGCGATTGTTTGTTCGTTGTCTGACAGCCGGGCAGATCTTGTCAGATCCAAAACAGCCTACGATCGCGCG
                                  -300                                                     -250

CCGGCTGGTTGCTTTTGGAAACGTAATCAGAAGCTTAAGGTGCCGGGTTAGACCTTGGCACGGCTGTTGCTGATAAGCGGCAGCAACACTGAGTGAGGG
                                  -200                                                     -150

CTGAGTGCACGCCGACGTGTAAGGCGAGCGATGCGCTCCTTCCCTTGAACCCGTGTGCCCCCGTTTCTGCGAGGGAAGCAAAGCTCGCAAAAGAAGCGC
                                   -100                                                     -50

GCAACGTTTGGCAAATCGGTTTGATGGAGAGCAGC ATG GCT TCA CTA AGA CAA ATC GCC TTC TAC GGG AAG GGC GGA ATC GGC
                                    Met Ala Ser Leu Arg Gln Ile Ala Phe Tyr Gly Lys Gly Gly Ile Gly
                                    1

AAG TCC ACC ACT TCG CAG AAC ACG CTA GCG GCG CTG GCA GAG ATG GGT CAG AAG ATC CTG ATT GTA GGG TGC GAT
Lys Ser Thr Thr Ser Gln Asn Thr Leu Ala Ala Leu Ala Glu Met Gly Gln Lys Ile Leu Ile Val Gly Cys Asp
            20                                                                          40

CCG AAA GCG GAC TCG ACT CGC CTT ATT CTG CAC GCC AAG GCT CAA GAC ACG ATT TTG AGT CTT GCC GCG AGC GCC
Pro Lus Ala Asp Ser Thr Arg Leu Ile Leu His Ala Lys Ala Gln Asp Thr Ile Leu Ser Leu Ala Ala Ser Ala
                                                                    60

GGC AGC GTG GAG GAT CTG GAG CTC GAG GAC GTA ATG AAG GTT GGC TAC CAG GAC ATT CGC TGC GTT GAG TCC GGT
Gly Ser Val Glu Asp Leu Glu Leu Glu Asp Val Met Lys Val Gly Try Gln Asp Ile Arg Cys Val Glu Ser Gly
                                                    80

GGC CCT GAG CCA GGT GTC GGC TGC GCC GGC CGC GGT GTC ATC ACC TCG ATC AAT TTT CTT GAA GAG AAC GGA GCC
Gly Pro Glu Pro Gly Val Gly Cys Ala Gly Arg Gly Val Ile Thr Ser Ile Asn Phe Leu Glu Glu Asn Gly Ala
                                100
```

# FIG. 2-2

TAC GAG AAC ATT GAC TAT GTT TCT TAC GAT GTG CTT GGC GAC GTT GTT TGC GGT GGC TTT GCG ATG CCA ATC CGC
Tyr Glu Asn Ile Asp Tyr Val Ser Try Asp Val Leu Gly Asp Val Val Cys Gly Gly Phe Ala Met Pro Ile Arg
120 140

GAA AAC AAG GCG CAG GAG ATC TAC ATC GTG ATG TCT GGT GAA ATG ATG GCA ATG TAT GCC GCA AAC AAT ATT TCC
Glu Asn Lys Ala Gln Glu Ile Tyr Ile Val Met Ser Gly Glu Met Met Ala Met Tyr Ala Ala Asn Asn Ile Ser
160

AAG GAA TTG GAA CTG GCG GAA GCG TTG GCC AAG AAG CTT GGC ACT CAA CTG ATC TAC TTC GTG CCG CGT GAC AAT
Lys Gly Ile Leu Lys Tyr Ala Asn Ser Gly Gly Val Arg Leu Gly Gly Leu Ile Cys Asn Glu Arg Gln Thr Asp
180

AAG GAA TTG GAA CTG GCG GAA GCG TTG GCC AAG AAG CTT GGC ACT CAA CTG ATC TAC TTC GTG CCG CGT GAC AAT
Lys Glu Leu Glu Leu Ala Glu Ala Leu Ala Lys Lys Leu Gly Thr Gln Leu Ile Tyr Phe Val Pro Arg Asp Asn
200

GTG GTG CAG CAT GCA GAG CTG CGT CGC ATG ACG GTG CTT GAA TAT GCA CCC GAT TCC AAG CAG GCT GAT CAC TAT
Val Val Gln His Ala Glu Leu Arg Arg Met Thr Val Leu Glu Tyr Ala Pro Asp Ser Lys Gln Ala Asp His Tyr
220 240

CGG AAA CTA GCG GCC AAG GTT CAC AAT AAT GGC GGC AAG GGC ATC ATT CCG ACC CCG ATC TCA ATG GAT GAG CTC
Arg Lys Leu Ala Ala Lys Val His Asn Asn Gly Gly Lys Gly Ile Ile Pro Thr Pro Ile Ser Met Asp Glu Leu
260

GAG GAC ATG CTG ATG GAG CAT GGC ATT ATA AAG GCC GTG GAT GAA TCA ATC ATC GGC AAA ACC GCC GCC GAA CTC
Glu Asp Met Leu Met Glu His Gly Ile Ile Lys Ala Val Asp Glu Ser Ile Ile Gly Lys Thr Ala Ala Glu Leu
280

GCA GCC TCG TAAAGGCCGCGGGTCGCCGCCTTGCGAAGGCGGCGACGATGCCGGTCTCCCTCACCCCCCTTCCCGGGGACCGG.........(3')
Ala Ala Ser

# FIG. 3

| | | | |
|---|---|---|---|
| TTT - Phe 5 (0.9) | TCT - Ser 0 (0.0) | TAT - Tyr 15 (2.9) | TGT - Cys 1 (0.1) |
| TTC - Rhe 14 (2.7) | TCC - Ser 7 (1.3) | TAC - Tyr 10 (1.9) | TGC - Cys 6 (1.1) |
| TTA - Leu 0 (0.0) | TCA - Ser 10 (1.9) | TAA - * 0 (0.0) | TGA - * 1 (0.1) |
| TTG - Leu 1 (0.1) | TCG - Ser 8 (1.5) | TAG - * 0 (0.0) | TGG - Trp 9 (1.7) |
| CTT - Leu 4 (0.7) | CCT - Pro 3 (0.5) | CAT - His 7 (1.3) | CGT - Arg 6 (1.1) |
| CTC - Leu 11 (2.1) | CCC - Pro 2 (0.3) | CAC - His 7 (1.3) | GCG - Arg 11 (2.1) |
| CTA - Leu 2 (0.3) | CCA - Pro 7 (1.3) | CAA - Gln 6 (1.1) | CGA - Arg 2 (0.3) |
| CTG - Leu 12 (2.3) | CCG - Pro 11 (2.1) | CAG - Gln 10 (1.9) | CGG 0 Arg 5 (0.9) |
| ATT - Ile 12 (2.3) | ACT - Thr 3 (0.5) | AAT - Asn 3 (0.5) | AGT - Ser 2 (0.3) |
| ATC - Ile 25 (4.8) | ACC - Thr 6 (1.1) | AAC - Asn 14 (2.7) | AGC - Ser 5 (0.9) |
| ATA - Ile 3 (0.5) | ACA - Thr 2 (0.3) | AAA - Lys 12 (2.3) | AGA - Arg 3 (0.5) |
| ATG - Met 12 (2.3) | ACG - Thr 7 (1.3) | AAG - Lys 23 (4.4) | AGG - Arg 5 (0.9) |
| GTT - Val 5 (0.9) | GCT - Ala 5 (0.9) | GAT - Asp 10 (1.9) | GGT - Gly 8 (1.5) |
| GTC - Val 10 (1.9) | GCC - Ala 9 (1.7) | GAC - Asp 24 (4.6) | GGC - Gly 30 (5.8) |
| GTA - Val 4 (0.7) | GCA - Ala 8 (1.5) | GAA - Glu 11 (2.1) | GGA - Gly 5 (0.9) |
| GTG - Val 16 (3.1) | GCG - Ala 11 (2.1) | GAG - Glu 23 (4.4) | GGG - Gly 7 (1.3) |

* - nonsense codon

# FIG. 4

```
5' - T T A G T G C T - C A T G A G A C C C T G G C A T G C C G G T T G C A A A G T C T T G G A T C A A G A A G...37bp...ATG.. Rj nifD
     A A G G T G C C G G G T T A G A C C T T G G C A C G G C T G T T G C T G A T A A G C G G C A G C A A C A C..143bp...ATG.. Rj nifH

     T A A T A A G C G C G G A C A G T G T T G G C A T G G C G A T T G C T G T T G A G T T G C A G C A A C A C..142bp...ATG.. RP nifH
     T A G T T T A T T T C A G A C G G C T G G C A C G A C T T T T G C A C G A T C A G C C C T G G G C G C G...62bp...ATG.. Rm nifH
     A T A C A T A A A C A G G C A C G G C T G G T A T G T T C C C T G C A C T T C T C T G C T G G C A A A C A...22bp...ATG.. Kp nifH
     G C G G T A G T G C A A A G C A A C C T G G C A C A G C C T T C G C A A T A C C C C T C C G A G A A C G C...35bp...ATG.. Kp nifF
     T C T G C A C A T C A C G C C G T A A G G G C G C A C G G T T T G C A T G G T T A T C A C C G T T C G G A...65bp...ATG.. Kp nifL
```

# FIG. 5

| Hybrid nif Plasmid | Vector | Physical Map |
|---|---|---|
| pSA31 | pBR322 | 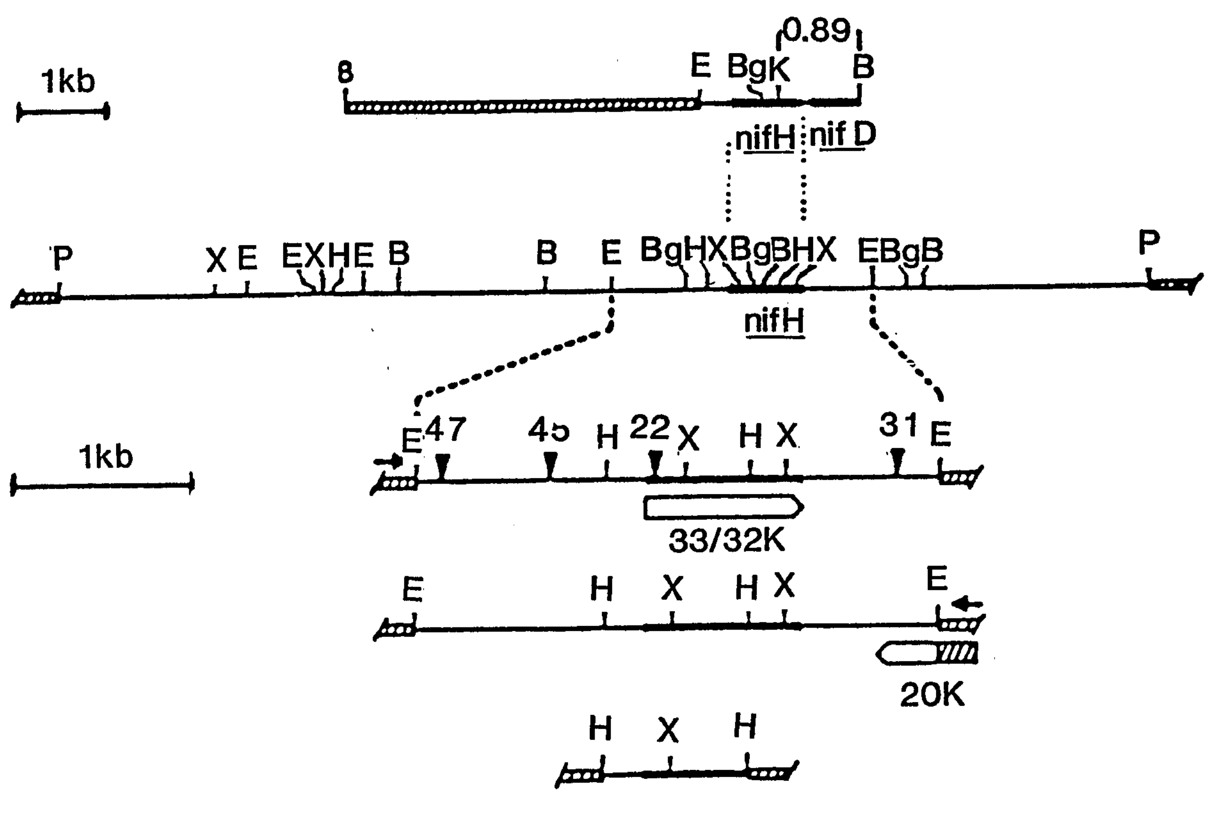 |
| pRJ7000 | pHE3 | |
| pRJ7003 | pACYC184 | |
| pRJ7004 | pACYC184 | |
| pRJ7001 | pACYC177 | |

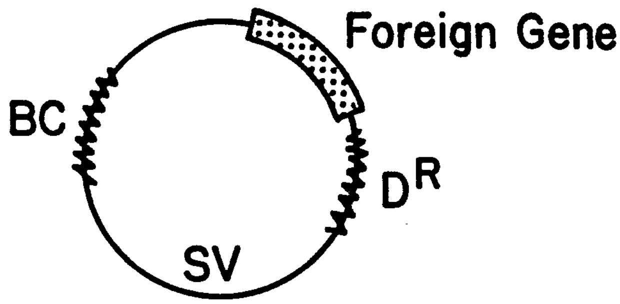


FIG. 6

Composite recombinant

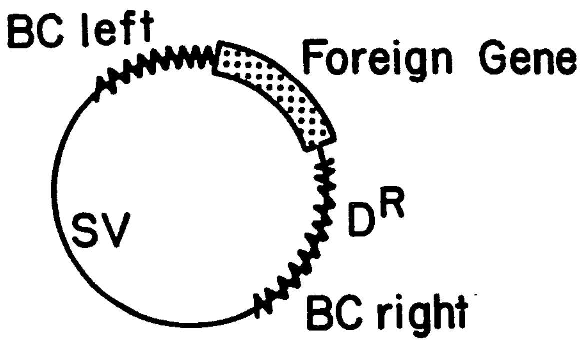


FIG. 7

Composite recombinant

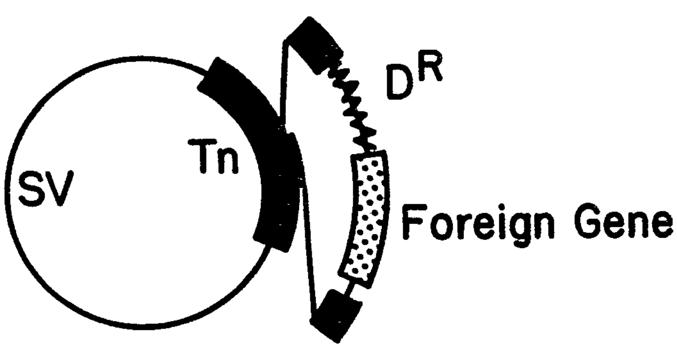


FIG. 8

Composite recombinant

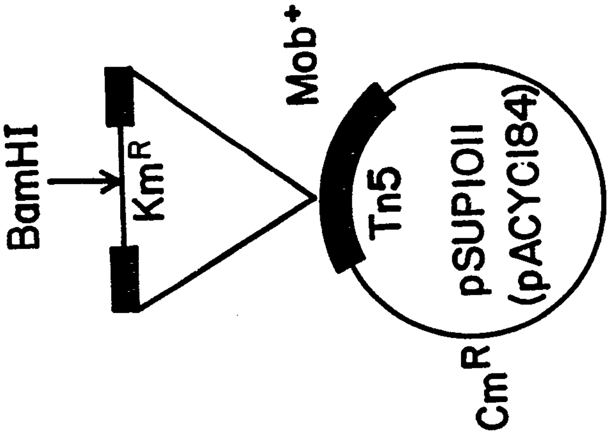
BamHI
Km^R
Mob+
Tn5
pSUP1011
(pACYC184)
Cm^R

FIG. 16

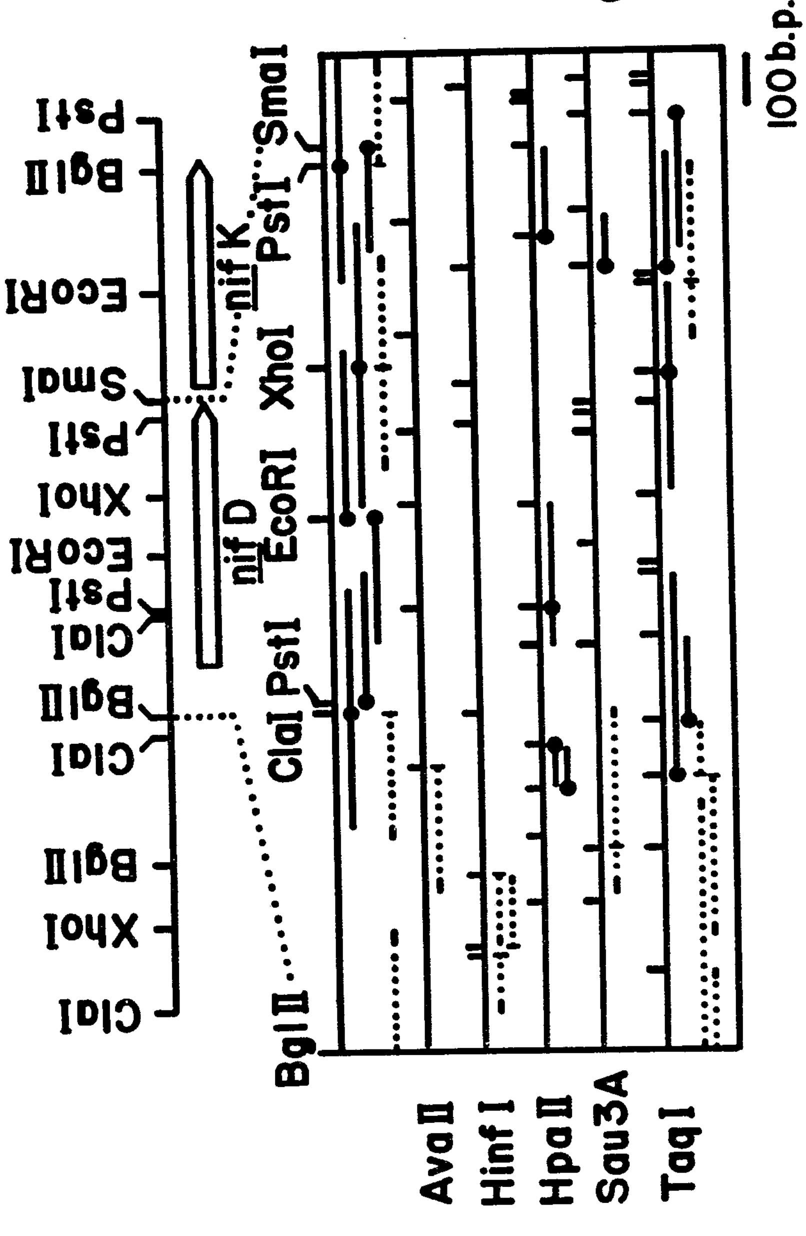
ClaI
XhoI
BglII
ClaI
BglII
ClaI
PstI
EcoRI
XhoI
PstI
SmaI
EcoRI
BglII
PstI
nif D
nif K
BglII
ClaI
PstI
EcoRI
XhoI
PstI
SmaI
AvaII
HinfI
HpaII
Sau3A
TaqI
100 b.p.

FIG. 9

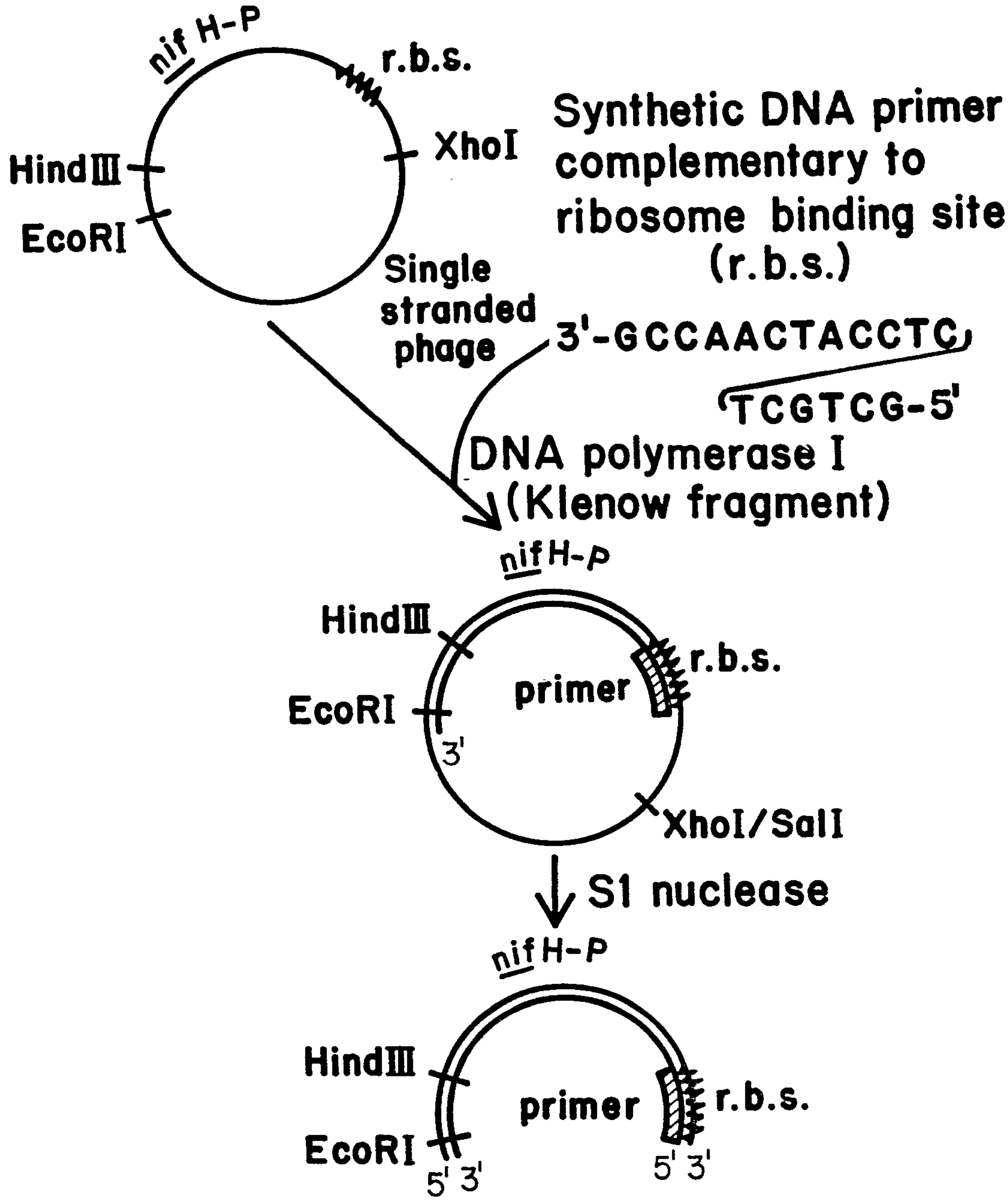


FIG. 10 nif H - promoter

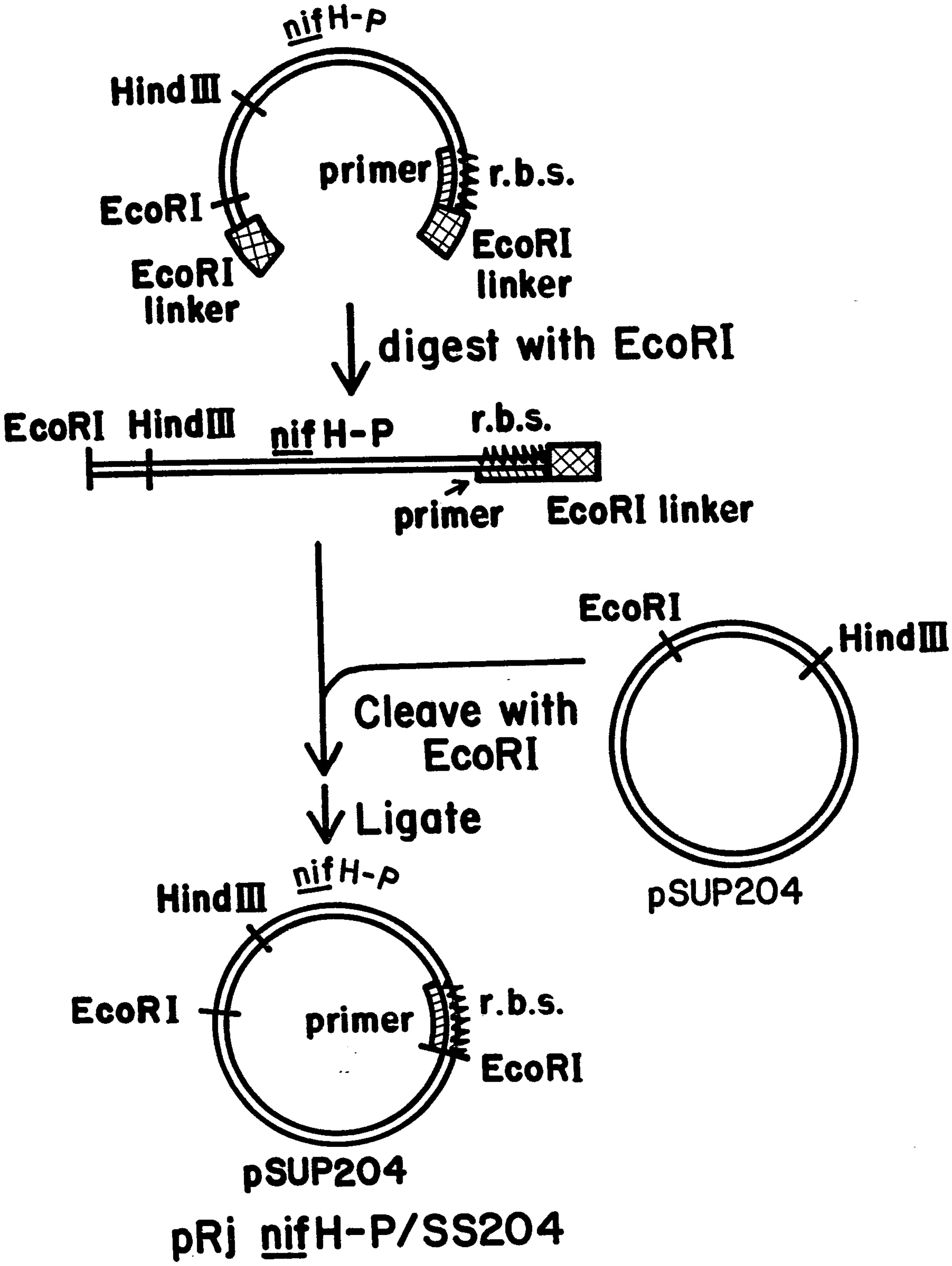
nifH-P
HindIII
primer
r.b.s.
EcoRI
EcoRI linker
EcoRI linker
digest with EcoRI
EcoRI HindIII nifH-P r.b.s.
primer EcoRI linker
EcoRI
HindIII
Cleave with EcoRI
Ligate
pSUP204
nifH-P
HindIII
EcoRI
primer
r.b.s.
EcoRI
pSUP204
pRj nifH-P/SS204

FIG. 11

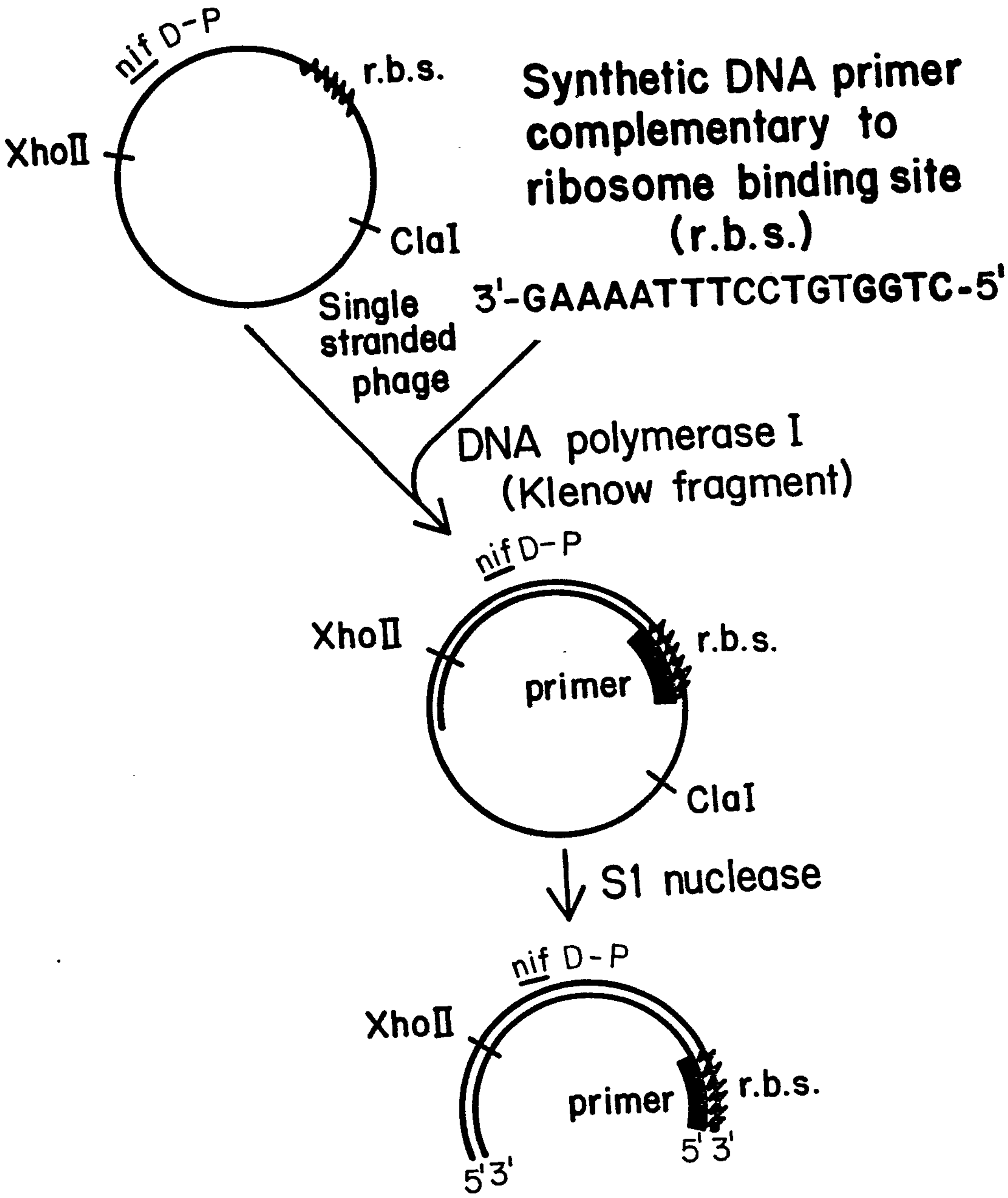


FIG. 12 nif D promoter

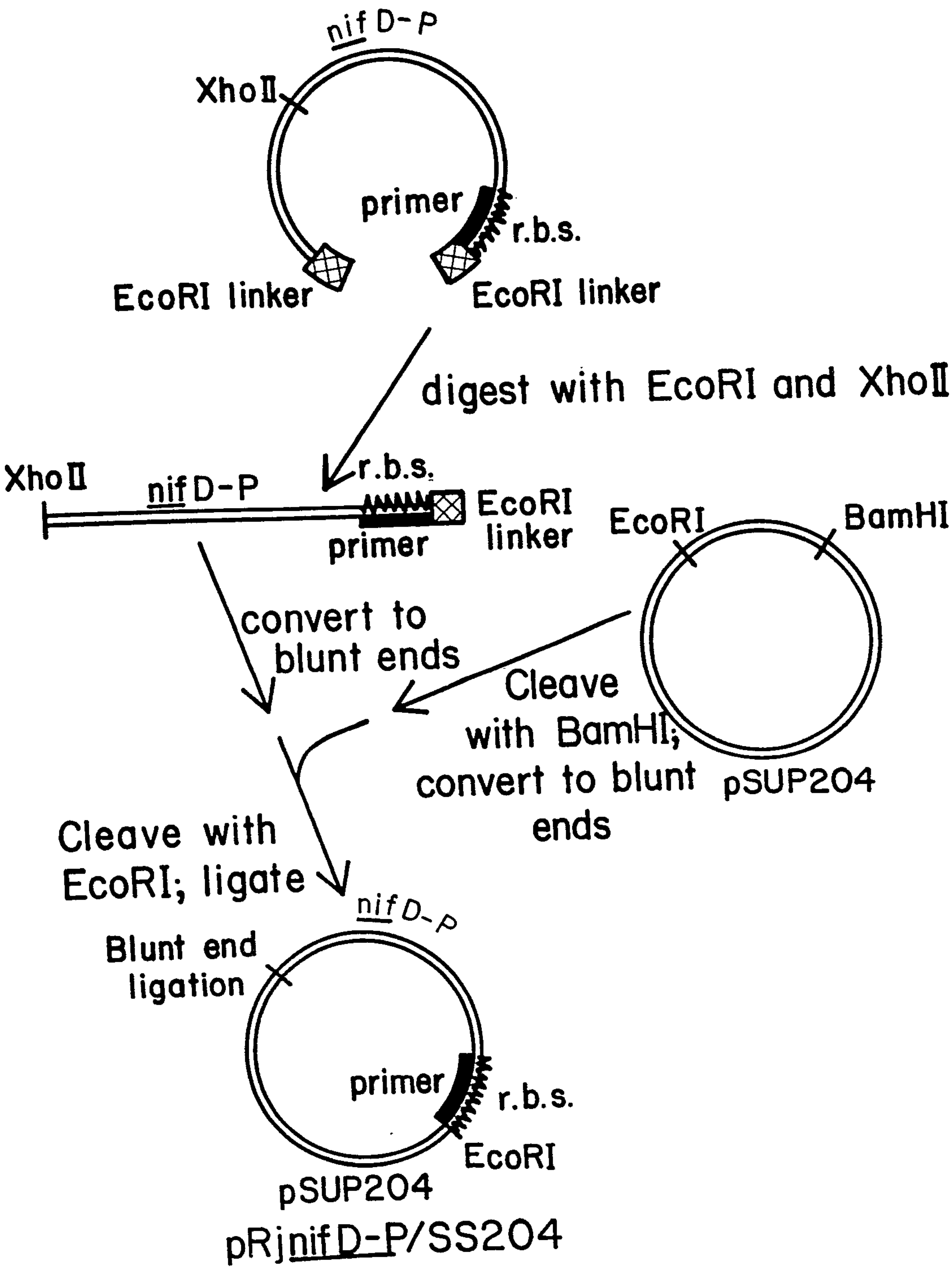

nif D-P
XhoII
primer
r.b.s.
EcoRI linker
EcoRI linker
digest with EcoRI and XhoII
XhoII
nif D-P
r.b.s.
EcoRI
linker
primer
EcoRI
BamHI
convert to
blunt ends
Cleave
with BamHI;
convert to blunt
ends
pSUP204
Cleave with
EcoRI; ligate
nif D-P
Blunt end
ligation
primer
r.b.s.
EcoRI
pSUP204
pRjnifD-P/SS204


FIG. 13

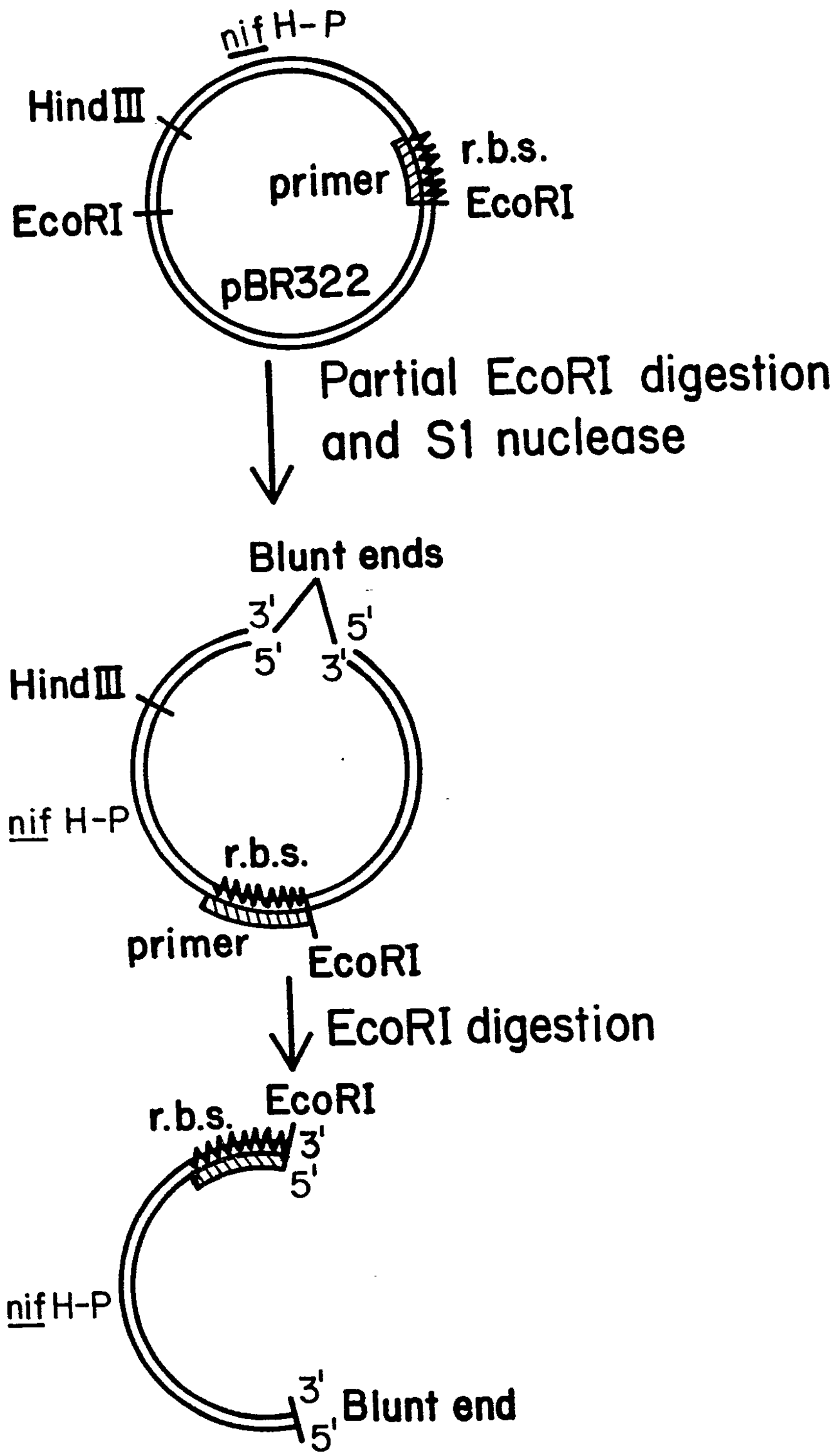
nif H-P
HindIII
r.b.s.
primer
EcoRI
EcoRI
pBR322
Partial EcoRI digestion
and S1 nuclease
Blunt ends
3'
5'
5'
3'
HindIII
nif H-P
r.b.s.
primer
EcoRI
EcoRI digestion
EcoRI
r.b.s.
3'
5'
nif H-P
3'
5'
Blunt end

FIG. 14

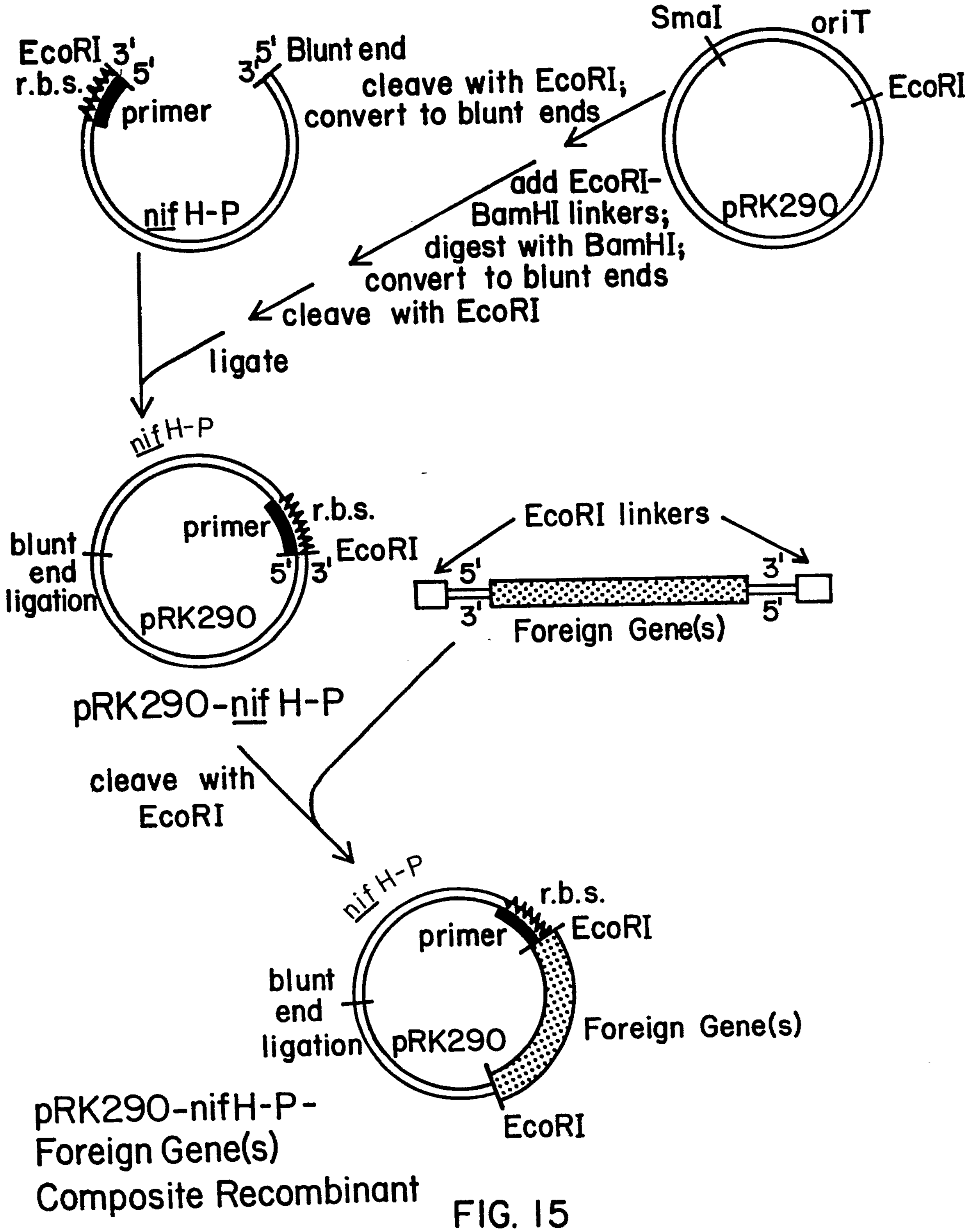

EcoRI 3' 5'
r.b.s.
primer
3' 5' Blunt end
nif H-P
SmaI
oriT
EcoRI
pRK290
cleave with EcoRI;
convert to blunt ends
add EcoRI-
BamHI linkers;
digest with BamHI;
convert to blunt ends
cleave with EcoRI
ligate
nif H-P
primer
r.b.s.
EcoRI
5' 3'
blunt
end
ligation
pRK290
pRK290-nif H-P
EcoRI linkers
5' 3'
3' 5'
Foreign Gene(s)
cleave with
EcoRI
nif H-P
r.b.s.
EcoRI
primer
blunt
end
ligation
pRK290
Foreign Gene(s)
EcoRI
pRK290-nifH-P-
Foreign Gene(s)
Composite Recombinant


FIG. 15